# EUROPEAN PATENT APPLICATION

(11) **EP 4 464 780 A1**
(43) Date of publication of application: **20.11.2024**
(21) Application number: 23739903.5
(22) Date of filing: 06.01.2023
(51) Int. Cl.: C12N 15/113, C12N 15/63, A61K 48/00, A61P 27/02, A61P 27/06

(54) **NUCLEOTIDE AND USE THEREOF**

(30) Priority: 11.01.2022 CN 202210025336
(71) Applicant: Guangzhou Reforgene Medicine Co., Ltd., Guangzhou, Guangdong 510535 (CN)
(72) Inventor: LIANG, Junbin, Guangzhou, Guangdong 510700 (CN); OU, Jiayu, Guangzhou, Guangdong 510700 (CN); XU, Hui, Guangzhou, Guangdong 510700 (CN); LIN, Simiao, Guangzhou, Guangdong 510700 (CN)
(74) Representative: dompatent von Kreisler Selting Werner - Partnerschaft von Patent- und Rechtsanwälten mbB
(86) International application number: PCT/CN2023/070867
(87) International publication number: WO 2023/134560

(57) **Abstract**

Provided are a nucleotide and a use thereof. Specifically provided is a nucleic acid molecule that specifically binds to exon 13 of USH2A pre-mRNA. Also provided is a preparation method for and use of the nucleic acid molecule. The nucleic acid molecule has the ability to specifically bind to sequence 3' of exon 13 of USH2A pre-mRNA or a fragment thereof, can perform targeted interference on Pre-mRNA splicing, and can increase the proportion of single skipping of exon 13 of USH2A pre-mRNA, so that safety can be ensured while efficiency is significantly improved.

## Description

### TECHNICAL FIELD

The present application relates to the field of biomedicine, in particular to a nucleotide and a use thereof.

### BACKGROUND

Usher syndrome is a genetic disease, also known as deafness-retinitis pigmentosa syndrome, characterized by various degrees of congenital sensorineural deafness and progressive vision loss due to retinitis pigmentosa (RP). Among them, USH2A gene mutations are the most common cause of type II Usher syndrome, covering more than 50% of patients with Usher syndrome. Meanwhile, mutations in the USH2A gene are also one of the important causes of non-syndromic retinitis pigmentosa (NSRP). Mutations in exon 13, exon 50, and intron 40 of the USH2A gene may trigger Usher syndrome. Over 1000 pathogenic mutations distributed throughout the USH2A gene have been identified so far, of which exon 13 is the most frequently mutated exon in the USH2A gene, accounting for about 35%. The USH2A coding region is about 15.6 kb in length. Current conventional gene therapy delivery methods (e.g., recombinant lentiviruses, recombinant adeno-associated viruses, etc.) are difficult to package such bulky coding sequences, making it difficult to treat by direct delivery of USH2A.

Therefore, there is an urgent need for a treatment method for USH2A gene mutation-related diseases that can improve the efficiency of exon skipping and ensure the safety of treatment in the art.

### CONTENT OF THE PRESENT INVENTION

In one aspect, the present application provides a nucleic acid molecule having the ability to specifically bind to the 3' sequence of exon 13 of USH2A pre-mRNA or a fragment thereof, wherein the genomic location corresponding to the 3' sequence of exon 13 of USH2A pre-mRNA is Chrl: 216246563-216246753.

In one aspect, the present application provides a nucleic acid molecule having the ability to specifically bind to the sequence of SEQ ID NO: 1 or a fragment thereof.

In one aspect, the present application provides a nucleic acid molecule complementary to 16 or more consecutive nucleotides of the sequence of SEQ ID NO: 1.

In one aspect, the present application provides a nucleic acid molecule comprising 16 or more consecutive nucleotides of the sequence of SEQ ID NO: 1 or a complementary sequence thereof.

In one aspect, the present application provides a gene expression cassette comprising or encoding the nucleotide sequence of the nucleic acid molecule as described in the present application, and optionally an expression regulatory element.

In one aspect, the present application provides a vector comprising or encoding the nucleotide sequence of the nucleic acid molecule as described in the present application, and/or the nucleotide sequence of the gene expression cassette as described in the present application.

In one aspect, the present application provides a viral particle comprising the nucleic acid molecule as described in the present application, the nucleotide sequence of the gene expression cassette as described in the present application, and/or the vector as described in the present application.

In one aspect, the present application provides a cell comprising the nucleic acid molecule as described in the present application, the nucleotide sequence of the gene expression cassette as described in the present application, the vector as described in the present application, and/or the viral particle as described in the present application.

In one aspect, the present application provides a pharmaceutical composition comprising the nucleic acid molecule as described in the present application, the nucleotide sequence of the gene expression cassette as described in the present application, the vector as described in the present application, the viral particle as described in the present application, and/or the cell as described in the present application, and optionally a pharmaceutically acceptable carrier.

In one aspect, the present application provides a kit comprising the nucleic acid molecule as described in the present application, the nucleotide sequence of the gene expression cassette as described in the present application, the vector as described in the present application, the viral particle as described in the present application, the cell as described in the present application, and/or the pharmaceutical composition as described in the present application.

In one aspect, the present application provides a method for preparing the nucleic acid molecule as described in the present application, comprising expressing and/or synthesizing the nucleic acid molecule capable of binding to the 3' sequence of exon 13 of USH2A or a fragment thereof, the genomic location of the 3' sequence of exon 13 of USH2A being Chrl: 216246563-216246753.

In one aspect, the present application provides a method for inhibiting the expression and/or functioning of exon 13 of USH2A pre-mRNA, comprising providing the nucleic acid molecule as described in the present application, the vector as described in the present application, the viral particle as described in the present application, the cell as described in the present application, the pharmaceutical composition as described in the present application, and/or the kit as described in the present application.

In one aspect, the present application provides a method for splice-skipping of exon 13 of USH2A pre-mRNA, comprising providing the nucleic acid molecule as described in the present application, the vector as described in the present application, the viral particle as described in the present application, the cell as described in the present application, the pharmaceutical composition as described in the present application, and/or the kit as described in the present application.

In one aspect, the present application provides a method for preparing a mature USH2A mRNA with a deletion of exon 13, comprising providing the nucleic acid molecule as described in the present application, the vector as described in the present application, the viral particle as described in the present application, the cell as described in the present application, the pharmaceutical composition as described in the present application, and/or the kit as described in the present application.

In one aspect, the present application provides a method for reducing the level of Usherin protein containing the expression product of exon 13, comprising providing the nucleic acid molecule as described in the present application, the vector as described in the present application, the viral particle as described in the present application, the cell as described in the present application, the pharmaceutical composition as described in the present application, and/or the kit as described in the present application.

In one aspect, the present application provides a method for preparing Usherin protein without the expression product of exon 13 and/or increasing the amount of Usherin protein without the expression product of exon 13, comprising providing the nucleic acid molecule as described in the present application, the vector as described in the present application, the viral particle as described in the present application, the cell as described in the present application, the pharmaceutical composition as described in the present application, and/or the kit as described in the present application.

In one aspect, the present application provides a method for restoring the function of a mutated Usherin protein, comprising providing the nucleic acid molecule as described in the present application, the vector as described in the present application, the viral particle as described in the present application, the cell as described in the present application, the pharmaceutical composition as described in the present application, and/or the kit as described in the present application.

In one aspect, the present application provides the use of the nucleic acid molecule as described in the present application, the vector as described in the present application, the viral particle as described in the present application, the cell as described in the present application, the pharmaceutical composition as described in the present application, and/or the kit as described in the present application in preparing a medicine for the prevention and/or treatment of diseases caused by USH2A gene mutations.

The present application provides a nucleic acid molecule having the ability to specifically bind to the 3' sequence of exon 13 of USH2A pre-mRNA or a fragment thereof. Currently, there are technologies for antisense oligonucleotides (AONs) targeting exon skipping. However, the existing AONs promote the co-skipping of exons 12 and 13 while promoting the skipping of exon 13. Some AON treatments even all result in double skipping of exons 12 and 13 instead of single skipping. However, due to the fact that exon 12 is 196 bp in full-length, not an integer multiple of 3, its deletion may result in frameshift mutations and the inactivation of USH2A protein. For example, the exon 12 of USH2A as described in the present application is exon 12 of human USH2A. For example, the exon 13 of USH2A as described in the present application is exon 13 of human USH2A. The oligonucleotides provided in the present application can perform targeted interference on pre-mRNA splicing and can increase the proportion of single skipping of exon 13, so that safety can be ensured while efficiency is significantly improved.

Other aspects and advantages of the present application will become readily apparent to those skilled in the art from the following detailed description. Only exemplary embodiments of the present application are shown and described in the following detailed description. As will be recognized by those skilled in the art, the contents of the present application enable those skilled in the art to make modifications to the specific embodiments disclosed without departing from the spirit and scope of the invention to which the present application pertains. Accordingly, the drawings and descriptions in the present application are merely exemplary, not limiting.

### BRIEF DESCRIPTION OF THE DRAWINGS

Specific features of the invention to which the present application pertains are set forth in the appended claims. The features and advantages of the invention to which the present application pertains will be better understood by reference to the exemplary embodiments and drawings described in detail below. The drawings are briefly described as follows:
Fig. 1 is a schematic diagram of the structure and function of U7-snRNA according to the present application.
Fig. 2 shows the effect of AONs targeting different target sites to induce the splice-skipping of exon 13 of USH2A pre-mRNA.
Figs. 3A-3B show the effect of U7 snRNAs targeting different target sites to induce the splice-skipping of exon 13 of USH2A pre-mRNA in reporter cells.
Figs. 4A-4B show the efficiency of U7 snRNA combinations targeting different target sites to induce the splice-skipping of exon 13 of USH2A pre-mRNA.
Fig. 5 is a schematic diagram of a snRNA vector with hnRNP A1.
Figs. 6A-6B show the efficiency of U7-hnRNP A1-snRNA to induce the splice-skipping of exon 13 of USH2A pre-mRNA.
Fig. 7 shows the efficiency of snRNAs targeting different targets to efficiently induce the single splice-skipping of exon 13 of USH2A pre-mRNA.

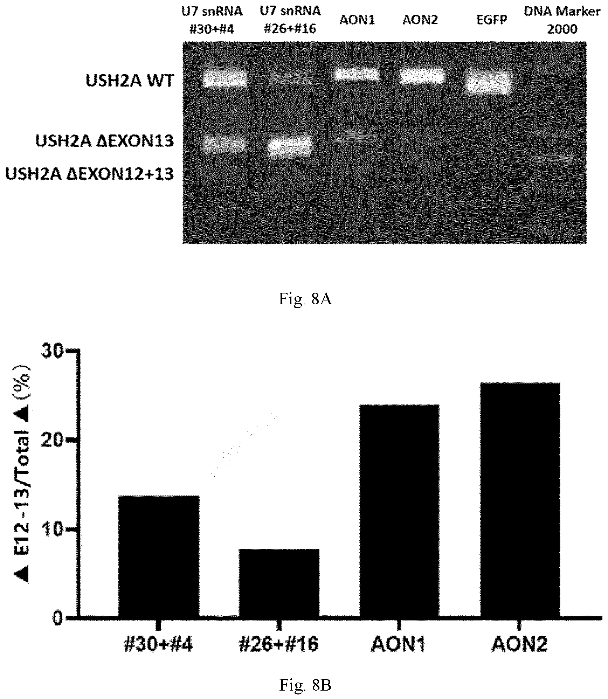

Fig. 8A shows the efficiency of chemically synthesized U7 snRNA to induce the splice-skipping of exon 13 of USH2A pre-mRNA detected in WERI cells. Lane 1: 50 pmol chemically synthesized and modified U7-snRNA#30-#4; lane 2: 50 pmol chemically synthesized and modified U7-snRNA#26-#16; lane 3: 50 pmol AON1; lane 4: 50 pmol AON2; lane 5: EGFP; lane 6: GL DNA Marker 2000. Fig. 8B is a histogram of quantitative analysis of RT-PCR bands. AE12-E13 represents USH2A mRNA splice-skipping both exons 12 and 13, and total A represents the sum of USH2A mRNA splice-skipping exon 13 and USH2A mRNA splice-skipping both exons 12 and 13.

### DETAILED DESCRIPTION OF THE PREFERRED EMBODIMENT

Other advantages and effects of the present invention will become readily apparent to those skilled in the art from the following description of specific embodiments.

### Definitions

In the present application, the term "isolated" generally refers to include a molecule isolated from other materials found in a natural source. For example, an isolated nucleic acid may refer to a nucleic acid molecule isolated from other materials found in a natural source. For example, an isolated molecule in the present application may be an artificial molecule, such as a synthetic molecule.

In the present application, the term "expression regulatory element" generally refers to a sequence provided for transcription and translation of a gene and/or for controlling the expression of a protein in vivo, e.g., in a desired host cell.

In the present application, the term "viral particle" generally refers to a nucleic acid vector. For example, a viral particle may be a viral vector that may be used to act as a nucleic acid delivery vehicle and comprises a vector genome (e.g., DNA and/or RNA) packaged within the viral particle.

In the present application, the term "antisense oligonucleotide" generally refers to a single-stranded oligonucleotide having a nucleobase sequence that permits hybridization to a corresponding fragment of a target nucleic acid.

In the present application, the term "nucleic acid" generally refers to a molecule composed of monomeric nucleotides. Nucleic acids include, but are not limited to, ribonucleic acids (RNAs), deoxyribonucleic acids (DNAs), single-stranded nucleic acids, double-stranded nucleic acids, small interfering ribonucleic acids (siRNAs), and micro RNAs (miRNAs).

In the present application, the term "small nuclear RNA" generally refers to a small nucleic acid molecule. For example, small nuclear RNAs may be involved in the processing of RNA in the nucleus of eukaryotes. For example, small nuclear RNAs may be combined with related proteins to form small nuclear ribonucleoproteins (snRNPs), which are involved in the splicing of messenger RNA precursors (pre-mRNAs).

In the present application, the term "complementary" or "base complementarity" generally refers to the ability of a nucleic acid base of an antisense oligonucleotide to undergo accurate base pairing (i.e., hybridization) with a corresponding nucleic acid base in a target nucleic acid, mediated by the binding of Watson-Crick interaction forces between the corresponding nucleic acid bases.

In the present application, the term "gene expression cassette" generally refers to a DNA segment that may be inserted into a nucleic acid or polynucleotide at a specific restriction site or by homologous recombination. For example, a gene expression cassette comprises a polynucleotide fragment encoding a nucleic acid of interest.

In the present application, the term "genomic location" generally refers to the chromosomal coordinates that describe the DNA region of interest. For example, chromosome coordinates may be consistent with the human genome database version Hg19 (alternatively also known as "Hg19 coordinates ") published in February 2009. For example, a DNA region of the present application may be derived from a region defined by Hg19 coordinates.

In the present application, the term "splice-skipping" generally means that the sequence of an RNA molecule that is splice-skipped during processing is not included in the processed RNA molecule. For example, a portion of the sequence may be skipped by splice-skipping. For example, a portion of the sequence may not be included in the spliced RNA molecule by splice-skipping.

In the present application, the term "modified oligonucleotide" generally refers to an oligonucleotide containing at least one modified internucleoside bond, modified sugar, and/or modified nucleobase. In the present application, the term "2'-O-methoxyethyl" (also known as 2'-MOE, 2'-OCH2CH2-OCH3, and MOE) generally refers to an O-methoxy-ethyl modification at the 2' position of a furanose ring. A 2'-O-methoxyethyl modified sugar is a modified sugar. In the present application, the term "2'-MOE nucleoside" (also known as 2'-O-methoxyethyl nucleoside) generally refers to a nucleoside containing a MOE-modified sugar moiety. In the present application, the term "2'-substituted nucleoside" generally refers to a nucleoside containing a substituent other than H or OH at the 2' position of a furanose ring. In certain embodiments, a 2'-substituted nucleoside includes a nucleoside with a bicyclic sugar modification. In the present application, the term "5-methylcytosine" generally refers to a cytosine modified with a methyl group attached to the 5' position. 5-Methylcytosine is a modified nucleobase.

In the present application, the term "modified phosphate bond" generally refers to a substitution or any changes in a modified phosphate bond relative to a naturally occurring internucleoside bond (i.e., a phosphodiester internucleoside bond).

In the present application, the term "stem-loop domain" generally refers to a secondary structure formed by a nucleic acid molecule itself through base pairing. For example, the double-stranded portion formed through base pairing is the "stem", and the sequence between paired bases forms the "loop".

In the present application, the term "consecutive" generally refers to two or more nucleobases immediately adjacent to each other.

In the present application, the term "exon" generally refers to a portion of a gene in the mature form of mRNA.

In the present application, the term "vector" generally refers to a nucleic acid molecule capable of transporting another nucleic acid to which it is linked.

In the present application, the term "Cas enzyme" generally refers to a CRISPR-related nuclease, a DNA endonuclease. For example, it may cause a double-strand break at a specific DNA sequence. Cas nucleases can generally be complementary to CRISPR sequences and can use the CRISPR sequences as a guide to recognize and cleave particular DNA strands.

In the present application, the term "pre-mRNA" generally refers to a precursor mRNA. For example, a primary transcript is a single-stranded ribonucleic acid product synthesized by DNA transcription, which may be unmodified. For example, the genomic structure of exons and introns in a precursor mRNA remains unchanged.

In the present application, the term "sm protein" generally refers to a protein or a variant thereof capable of binding to snRNAs to form a small nuclear ribonucleoprotein complex snRNP.

In the present application, the term "USH2A" generally refers to a gene encoding the Usherin protein. For example, the NCBI gene accession number for USH2A (Usher Syndrome Type-2A) can be 7399. In the present application, USH2A may encompass its unprocessed form, any processed form, variants thereof, or substances comprising functionally active fragments thereof.

In the present application, the term "administering" refers generally to providing a pharmaceutical agent to an animal and includes, but is not limited to, administration by a medical professional and self-administration.

### Detailed description of the invention

In one aspect, the present application provides a nucleic acid molecule. The nucleic acid molecule may have the ability to specifically bind to 3' sequence of exon 13 of USH2A pre-mRNA or a fragment thereof. The genomic location corresponding to the 3' sequence of exon 13 of USH2A pre-mRNA is Chrl: 216246563-216246753 (corresponding to NCBI database version GRch38). For example, the genomic location corresponding to the sequence of the 3' sequence of exon 13 of USH2A pre-mRNA of the present application is Chr1: 216246563-216246626, Chrl: 216246563-216246621, Chrl: 216246563-216246624, Chrl: 216246563-216246617, Chrl: 216246563-216246591, Chrl: 216246563-216246596, Chrl: 216246563-216246593, Chrl: 216246563-216246590, and/or Chrl: 216246563-216246586. For example, the genomic location corresponding to the 3' sequence of exon 13 of USH2A pre-mRNA as described in the present application is Chrl: 216246563-216246626, chrl: 216246567-216246626, chrl: 216246570-216246626, chrl: 216246594-216246626, chrl: 216246598-216246626, and/or Chrl: 216246603-216246626. For example, the genomic location corresponding to the 3' sequence of exon 13 of USH2A pre-mRNA as described in the present application is Chrl: 216246563-216246621, chrl: 216246567-216246621, chrl: 216246570-216246621, chrl: 216246594-216246621, chrl: 216246598-216246621, and/or Chrl: 216246603-216246621. For example, the genomic location corresponding to the 3' sequence of exon 13 of USH2A pre-mRNA as described in the present application is Chr1: 216246563-216246624, chrl: 216246567-216246624, chrl: 216246570-216246624, chrl: 216246594-216246624, chrl: 216246598-216246624, and/or Chrl: 216246603-216246624. For example, the genomic location corresponding to the 3' sequence of exon 13 of USH2A pre-mRNA as described in the present application is Chrl: 216246563-216246617, chrl: 216246567-216246617, chrl: 216246570-216246617, chrl: 216246594-216246617, chrl: 216246598-216246617, and/or Chrl: 216246603-216246617. For example, the genomic location corresponding to the 3' sequence of exon 13 of USH2A pre-mRNA as described in the present application is Chrl: 216246563-216246593, chrl: 216246567-216246593, and/or Chrl: 216246570-216246593. For example, the genomic location corresponding to the 3' sequence of exon 13 of USH2A pre-mRNA as described in the present application is Chr1: 216246563-216246626, and its sequence may be shown as SEQ ID NO: 39. For example, it may be a binding of 70%, 80%, 90%, 95%, 98%, 99% or more base pairs. For example, it may be a binding to a mutated sequence. For example, the binding may comprise a 1 bp, 2 bp, 3 bp, 4 bp, or 5 bp mismatch.

Exon 13 of the USH2A gene may contain mutations including c.2802T>G (p.Cys934Trp, the highest frequency mutation in Chinese patients), c.2299delG (p.E767SfsX21, the highest frequency mutation in European and American patients), c.2276G>T (amino acid change: p.C759F), C.2522C>A (p.S841Y), c.2242C>T (p.Gln748X), c.2541C>A (C847X), c.2761delC (Leu921fs) and C.2776C>T (p.R926C), C.2209C>T, C.2310delA, c.2391_2392deITG, c.2431A>T, C.2431_2432delAA, c.2440C>T, c.2525dup, C.2610C>A, C.2755C>T, C.2176T>C, C.2236C>G, c.2296T>C, and/or C.2332G>T.

In an aspect, the present application provides a nucleic acid molecule. The nucleic acid molecule may have the ability to specifically bind to the sequence of SEQ ID NO: 1 or a fragment thereof. For example, it may be a binding of 70%, 80%, 90%, 95%, 98%, 99% or more base pairs. For example, it may be a binding to a mutated sequence. For example, the binding may comprise a 1 bp, 2 bp, 3 bp, 4 bp, or 5 bp mismatch.

In one aspect, the present application provides a nucleic acid molecule. The nucleic acid molecule may be complementary to 16 or more consecutive nucleotides of a sequence of SEQ ID NO: 1. For example, it may be complementary of 70%, 80%, 90%, 95%, 98%, 99% or more base pairs. For example, it may be complementary to a mutated sequence. For example, the complementary may comprise a 1 bp, 2 bp, 3 bp, 4 bp, or 5 bp mismatch.

In one aspect, the present application provides a nucleic acid molecule. The nucleic acid molecule may comprise 16 or more consecutive nucleotides of a sequence of SEQ ID NO: 1 or a complementary sequence thereof. For example, it may be complementary of 70%, 80%, 90%, 95%, 98%, 99% or more base pairs. For example, it may be complementary to a mutated sequence. For example, the complementary may comprise a 1 bp, 2 bp, 3 bp, 4 bp, or 5 bp mismatch.

For example, the nucleic acid molecule comprises at least 16 nucleotides that may be complementary to the 3' sequence of exon 13 of USH2A pre-mRNA or the fragment thereof. For example, the nucleic acid molecule comprises 22 to 27 nucleotides that may be complementary to the 3' sequence of exon 13 of USH2A pre-mRNA or the fragment thereof. For example, the nucleic acid molecule comprises 15 to 27, 16 to 27, 18 to 27, 20 to 27, 22 to 27, 25 to 27, 15 to 25, 16 to 25, 18 to 25, 20 to 25, 22 to 25, 15 to 22, 16 to 22, 18 to 22, 20 to 22, 15 to 20, 16 to 20, 18 to 20, 15 to 18, or 16 to 18 nucleotides that may be complementary to the 3' sequence of exon 13 of USH2A pre-mRNA or a fragment thereof. For example, the recognition domain sequence may be 16 bp or more in length, further 18 bp-40 bp in length, and still further 20 bp-27 bp in length. In some embodiments of chemically synthesized and modified U7 snRNA, the length of the recognition domain sequence may be further extended along the 5' or/and 3' ends of a target sequence by reverse complementary pairing, preferably the length of the extended single recognition domain sequence is less than or equal to 40bp.

For example, the nucleic acid molecule may comprise at least 16 nucleotides. For example, the nucleic acid molecule may comprise 22 to 27 nucleotides. For example, the nucleic acid molecule may comprise 15 to 27, 16 to 27, 18 to 27, 20 to 27, 22 to 27, 25 to 27, 15 to 25, 16 to 25, 18 to 25, 20 to 25, 22 to 25, 15 to 22, 16 to 22, 18 to 22, 20 to 22, 15 to 20, 16 to 20, 18 to 20, 15 to 18, or 16 to 18 nucleotides. For example, the nucleic acid molecule is an AON molecule.

For example, the nucleic acid molecule may comprise at least 60 nucleotides. For example, the nucleic acid molecule may comprise at least 65, at least 70, at least 80, at least 90, at least 100, at least 150, at least 200, or 500 nucleotides. For example, the nucleic acid molecule is a snRNA molecule. For example, a snRNA molecule may comprise more elements and have a theoretically infinite length. In some embodiments, a chemically synthesized snRNA sequence has a total length greater than or equal to 96 bp. In some embodiments, 3-40 bases flanking a chemically synthesized snRNA are modified and linked via specific phosphate bonds.

For example, the nucleic acid molecule may comprise a sequence complementary to a sequence corresponding to genomic location selected from the following group: Chrl: 216246603-216246626, Chrl: 216246598-216246621, Chrl: 216246598-216246624, Chrl: 216246594-216246617, Chrl: 216246570-216246593, Chrl: 216246570-216246591, Chrl: 216246570-216246596, Chrl: 216246570-216246593, Chrl: 216246570-216246596, Chrl: 216246567-216246590, and Chrl: 216246563-216246586.

For example, the nucleic acid molecule may comprise a sequence complementary to any of SEQ ID NOs: 10-21. For example, it may be complementary of 70%, 80%, 90%, 95%, 98%, 99% or more base pairs. For example, it may be complementary to a mutated sequence. For example, the complementary may comprise a 1 bp, 2 bp, 3 bp, 4 bp, or 5 bp mismatch. In some embodiments of chemically synthesized and modified U7 snRNA, there may be 0-5 mismatched nucleotides, e.g., 0-1, in the reverse complementary pairing between a recognition domain and a target site.

For example, the nucleic acid molecule may comprise a sequence of any one of SEQ ID NOs: 10-21.

For example, the nucleic acid molecule may comprise a modified nucleotide.

For example, the modified nucleotide may comprise a modification selected from the following group: 2'-O-alkyl, 2'-O-methoxy, and/or 2'-O-methoxyethyl.

For example, the modified nucleotide may comprise a modification selected from the following group: 2'-O-methyl and/or 2'-O-ethyl.

For example, the nucleic acid molecule may comprise at least one modified nucleotide. In some embodiments, only 1-10, 6-80, or all of the nucleotides flanking a snRNA are modified, which is a combination of one or more modifications, and linked via specific phosphate bonds, which are a combination of one or more phosphate bonds. In some embodiments, all nucleotides in the chemically synthesized U7 snRNA are linked to each other via phosphorothioate bonds and modified with a 2'-O-methoxy group. In some embodiments, only 3 nucleotides flanking a snRNA are linked via phosphorothioate bonds and modified with a 2'-O-methoxy group. In some preferred embodiments of chemically synthesized and modified U7 snRNA, the first nucleotide at the 5' end of U7 snRNA is preferably adenylate (A). If the first nucleotide at the 5' end of the recognition domain is not adenylate (A), adenylate (A) is linked at the 5' end.

For example, the 5' end of the nucleic acid molecule may comprise at least one modified nucleotide. For example, the 5' end of the nucleic acid molecule may comprise 1 to 3 modified nucleotides. For example, the 5' end of the nucleic acid molecule may comprise 1, 2, 3, 4, or 5 modified nucleotides.

For example, the 3' end of the nucleic acid molecule may comprise at least one modified nucleotide. For example, the 3' end of the nucleic acid molecule may comprise 1 to 3 modified nucleotides. For example, the 3' end of the nucleic acid molecule may comprise 1, 2, 3, 4, or 5 modified nucleotides.

For a nucleic acid molecule as described in the present application, the nucleotide molecules of the nucleic acid molecule may be selected from the following group: 6'-modified bicyclic nucleosides, 5'-modified bicyclic nucleosides, 6'-disubstituted bicyclic nucleosides, tetrahydropyran nucleoside analogs, and/or 2'-deoxy 2'-fluoro-β-D-arabinose nucleotides (2'-FANA modified nucleotides).

For example, the nucleic acid molecule has modified phosphate bonds.

For example, the nucleotide molecules are linked via phosphate bonds selected from the following group: thiophosphate linkage, phosphorodithioate linkage, phosphotriester linkage, alkyl phosphonate linkage, aminoalkyl phosphotriester linkage, alkylene phosphonate linkage, phosphinate linkage, phosphoramidate linkage and aminoalkyl phosphoramidate linkage, thiophosphoramidate linkage, thiocarbonylalkyl phosphonate linkage, thiocarbonylalkyl phosphotriester linkage, phosphorothioate linkage, selenophosphate linkage, and/or boranophosphate linkage.

For example, the modified phosphate bonds may be selected from the following group: phosphodiester bond, phosphotriester bond, phosphorothioate bond (5'O-P(S)O-3O-, 5'S-P(O)O-3'-O-, and 5'O-P(O)O-3'S-), phosphorodithioate bond, Rp-phosphorothioate bond, Sp-phosphorothioate bond, boranophosphate bond, methylene bond (methylimino), amide bond (3'-CH₂-CO-NH-5' and 3'-CH₂-NH-CO-5'), methylphosphonate bond, 3'-thiomethylal bond (3'S-CH₂-O5'), amide bond (3'CH₂-C(O)NH-5'), and/or phosphoramidate group.

For example, the modified phosphate bonds may be selected from the following group: thiophosphate bond, phosphorodithioate bond, alkyl phosphate bond, amide phosphate bond, and/or boranophosphate bond.

For example, the modified phosphate bonds may be selected from the following group: phosphodiester bond, phosphotriester bond, phosphorothioate bond (5'O-P(S)O-3O-, 5'S-P(O)O-3'-O-, and 5'O-P(O)O-3'S-), phosphorodithioate bond, Rp-phosphorothioate bond, Sp-phosphorothioate bond, boranophosphate bond, methylene bond (methylimino), amide bond (3'-CH₂-CO-NH-5' and 3'-CH₂-NH-CO-5'), alkyl phosphate bond, amide phosphate bond, methyl phosphonate bond, 3'-thiomethylal bond, (3'S-CH₂-O5'), amide bond (3'CH₂-C(O)NH-5'), and/or phosphoramidate group.

For example, the nucleic acid molecule may comprise at least one modified phosphate bond.

For example, the 5' end of the nucleic acid molecule may comprise at least one modified phosphate bond. For example, the 5' end of the nucleic acid molecule may comprise 1 to 3 modified phosphate bonds. For example, the 5' end of the nucleic acid molecule may comprise 1, 2, 3, 4, or 5 modified phosphate bonds.

For example, the 3' end of the nucleic acid molecule may comprise at least one modified phosphate bond. For example, the 3' end of the nucleic acid molecule may comprise 1 to 3 modified phosphate bonds. For example, the 5' end of the nucleic acid molecule may comprise 1, 2, 3, 4, or 5 modified phosphate bonds.

For example, the nucleic acid molecule may comprise DNA and/or RNA.

For example, the nucleic acid molecule is single-stranded or double-stranded.

For example, the nucleic acid molecule may comprise an antisense oligonucleotide, shRNA, siRNA, miRNA, and/or aptamer.

For example, the nucleic acid molecule may comprise small nuclear RNA.

For example, the small nuclear RNA may comprise U1 small nuclear RNA and/or U7 small nuclear RNA. For example, the small nuclear RNA may comprise U1, U2, U3, U4, U5, U6, and/or U7 small nuclear RNA. For example, the small nuclear RNA may comprise U1 small nuclear RNA. For example, the small nuclear RNA may comprise U7 small nuclear RNA.

For example, the nucleic acid molecule does not bind to a Cas enzyme. For example, the nucleic acid molecule may not comprise a structure capable of binding to a Cas enzyme. For example, the Cas enzyme is a Cas13 enzyme.

For example, the nucleic acid molecule may comprise a stem-loop domain or a derivative structure thereof. For example, the stem-loop domain may comprise the sequence of SEQ ID NO: 6.

For example, the nucleic acid molecule may comprise a domain capable of binding to sm protein or a derivative structure thereof. For example, the domain capable of binding to sm protein may comprise the sequence of SEQ ID NO: 5. For example, the domain capable of binding to sm protein may comprise an optimized smOPT sequence. For example, the domain binding to sm protein may be human-derived, murine-derived, or porcine-derived.

For example, the nucleic acid molecule may comprise a domain capable of recruiting splicing regulatory proteins. For example, the nucleic acid molecule may comprise a domain capable of binding to proteins selected from the following group: SRSF1 (Serine And Arginine Rich Splicing Factor 1), RBM4 (RNA Binding Motif Protein 4), DAZAP1 (DAZ Associated Protein 1), and SR (Serine And Arginine-Rich Protein).

For example, the nucleic acid molecule may comprise a domain capable of binding to the hnRNP A1 protein. For example, the domain capable of binding to hnRNP A1 protein may comprise the sequence of SEQ ID NO: 30.

For example, the nucleic acid molecule of the present application has the ability to specifically bind to the 3' sequence of exon 13 of USH2A pre-mRNA or a fragment thereof, the genomic location corresponding to the 3' sequence of exon 13 of USH2A pre-mRNA being Chr1: 216246563-216246753. The nucleic acid molecule comprises at least 16 nucleotides that may be complementary to the 3' sequence of exon 13 of USH2A pre-mRNA or a fragment thereof. The nucleic acid molecule may comprise at least 16 nucleotides.

For example, the nucleic acid molecule of the present application has the ability to specifically bind to the 3' sequence of exon 13 of USH2A pre-mRNA or a fragment thereof, the genomic location corresponding to the 3' sequence segment of exon 13 of USH2A pre-mRNA being Chrl: 216246563-216246753. The nucleic acid molecule comprises at least 16 nucleotides that may be complementary to the 3' sequence of exon 13 of USH2A pre-mRNA or a fragment thereof. The nucleic acid molecule may comprise at least 60 nucleotides.

For example, the nucleic acid molecule of the present application has the ability to specifically bind to the 3' sequence of exon 13 of USH2A pre-mRNA or a fragment thereof, the genomic location corresponding to the 3' sequence of exon 13 of USH2A pre-mRNA being Chr1: 216246563-216246753. The nucleic acid molecule comprises at least 16 nucleotides that may be complementary to the 3' sequence of exon 13 of USH2A pre-mRNA or a fragment thereof. The nucleic acid molecule may comprise at least 60 nucleotides, and 3-40 bases flanking the chemically synthesized snRNA are modified and linked via specific phosphate bonds.

For example, the nucleic acid molecule of the present application has the ability to specifically bind to the 3' sequence of exon 13 of USH2A pre-mRNA or a fragment thereof, the genomic location corresponding to the 3' sequence of exon 13 of USH2A pre-mRNA being Chr1: 216246563-216246753. The nucleic acid molecule comprises at least 16 nucleotides that may be complementary to the 3' sequence of exon 13 of USH2A pre-mRNA or a fragment thereof. The nucleic acid molecule may comprise at least 60 nucleotides, and 3-40 bases flanking the chemically synthesized snRNA are modified with a 2'-methoxy group and linked via phosphorothioate bonds.

For example, the nucleic acid molecule of the present application has the ability to specifically bind to the 3' sequence of exon 13 of USH2A pre-mRNA or a fragment thereof, the genomic location corresponding to the 3' sequence of exon 13 of USH2A pre-mRNA being Chr1: 216246563-216246753. The nucleic acid molecule comprises at least 16 nucleotides that may be complementary to the 3' sequence of exon 13 of USH2A pre-mRNA or a fragment thereof. The nucleic acid molecule may comprise at least 60 nucleotides, and 3 bases flanking chemically synthesized snRNA are modified with a 2'-methoxy group and linked via phosphorothioate bonds. The nucleic acid molecule may comprise a domain capable of binding to sm protein or a derivative structure thereof. Optionally, the nucleic acid molecule may comprise a domain capable of binding to the hnRNP A1 protein.

In an aspect, the present application provides a gene expression cassette. The gene expression cassette may comprise or encode the nucleotide sequence of a nucleic acid molecule as described in the present application, and optionally an expression regulatory element.

In an aspect, the present application provides a vector. The vector may comprise or encode the nucleotide sequence of a nucleic acid molecule as described in the present application, and/or the nucleotide sequence of a gene expression cassette as described in the present application. In an aspect, the present application provides a viral particle. The viral particle may comprise a nucleic acid molecule as described in the present application, the nucleotide sequence of a gene expression cassette as described in the present application, and/or a vector as described in the present application. For example, the vector of the present application may be pUC57. The vector may also be selected from the following group: pAAV-CMV (TAKARA Corporation, Code No. 6650), lentiviruses, transposons, and nucleic acid vectors known in the art. For example, in the present application, U7 snRNA may be delivered by AAV to induce the splice-skipping of exon 13 of USH2A pre-mRNA. The capsid protein of the AAV may be naturally derived, or a variant based on naturally-derived capsid protein, or undergo directed evolution or reasonable amino acid/peptide modification (codon optimization, chimerism of different serotype functional peptide segments, etc.) to improve the characteristics such as tissue and organ tropism, immunogenicity, and transfection efficiency, e.g., AAV2.5, AAV 2i8, AAV-TT, AAV9.HR, and CAM130. The naturally derived AAV capsid protein may be derived from animals or plants. The animal-derived AAV capsid protein may be derived from humans (such as AAV1, AAV2, AAV3, AAV5, AAV6, AAV7, AAV8, and AAV9), non-human primates (such as AAVrh.8, AAVrh.10, and AAVrh.43), vertebrates such as mouse and pig, or insects. In the AAV plasmid system of the present invention, for example, the AAV ITR serotype should be identical to the Rep gene serotype and may be different from the Cap gene serotype.

In an aspect, the present application provides a cell. The cell may comprise a nucleic acid molecule as described in the present application, the nucleotide sequence of a gene expression cassette as described in the present application, a vector as described in the present application, and/or a viral particle as described in the present application.

In an aspect, the present application provides a pharmaceutical composition. The pharmaceutical composition may comprise a nucleic acid molecule as described in the present application, the nucleotide sequence of a gene expression cassette as described in the present application, a vector as described in the present application, a viral particle as described in the present application, and/or a cell as described in the present application, and optionally a pharmaceutically acceptable carrier.

For example, the pharmaceutical composition may comprise a first vector that may comprise a nucleic acid molecule as described in the present application, and a second vector that may comprise a nucleic acid molecule that may have the ability to specifically bind to exon 13 of USH2A pre-mRNA and flanking regions or a fragment thereof. For example, the second vector may comprise a nucleic acid molecule having the ability to specifically bind to a sequence selected from intron 12-exon 13-intron 13 of USH2A pre-mRNA. For example, the sequence may be selected from exon 13 and flanking adjacent target regions. For example, the sequence may be selected from the pre-mRNA region corresponding to chrl: 216246563-216247246. For example, the sequence may be a combination of two or more U7-snRNAs targeting not identical target sequences.

For example, the flanking regions of exon 13 of USH2A pre-mRNA may comprise intron 12 and/or intron 13 of USH2A pre-mRNA.

In an aspect, the present application provides a kit. The kit may comprise a nucleic acid molecule as described in the present application, the nucleotide sequence of a gene expression cassette as described in the present application, a vector as described in the present application, a viral particle as described in the present application, a cell as described in the present application, and/or a pharmaceutical composition as described in the present application.

In an aspect, the present application provides a method for preparing a nucleic acid molecule as described in the present application. The method may comprise expressing and/or synthesizing the nucleic acid molecule capable of binding to the 3' sequence of exon 13 of USH2A or a fragment thereof, the genomic location of the 3' sequence of exon 13 of USH2A being Chrl: 216246563-216246753.

In an aspect, the present application provides a method for inhibiting the expression and/or function of exon 13 of USH2A pre-mRNA. The method may comprise providing a nucleic acid molecule as described in the present application, a vector as described in the present application, a viral particle as described in the present application, a cell as described in the present application, a pharmaceutical composition as described in the present application, and/or a kit as described in the present application. For example, the inhibition of the expression and/or function of exon 13 of USH2A pre-mRNA may refer to allowing the mature USH2A mRNA to not contain the region of the expression product of exon 13.

In an aspect, the present application provides a method for splice-skipping of exon 13 of USH2A pre-mRNA. The method may comprise providing a nucleic acid molecule as described in the present application, a vector as described in the present application, a viral particle as described in the present application, a cell as described in the present application, a pharmaceutical composition as described in the present application, and/or a kit as described in the present application.

In an aspect, the present application provides a method for preparing a mature USH2A mRNA with a deletion of exon 13. The method may comprise providing a nucleic acid molecule as described in the present application, a vector as described in the present application, a viral particle as described in the present application, a cell as described in the present application, a pharmaceutical composition as described in the present application, and/or a kit as described in the present application.

In an aspect, the present application provides a method for reducing the level of Usherin protein that may contain the expression product of exon 13. The method may comprise providing a nucleic acid molecule as described in the present application, a vector as described in the present application, a viral particle as described in the present application, a cell as described in the present application, a pharmaceutical composition as described in the present application, and/or a kit as described in the present application.

In an aspect, the present application provides a method for preparing Usherin protein without the expression product of exon 13 and/or increasing the amount of Usherin protein without the expression product of exon 13. The method may comprise providing a nucleic acid molecule as described in the present application, a vector as described in the present application, a viral particle as described in the present application, a cell as described in the present application, a pharmaceutical composition as described in the present application, and/or a kit as described in the present application. For example, the Usherin protein prepared in the present application does not contain a polypeptide expressed by exon 13 or a mutant thereof.

In an aspect, the present application provides a method for restoring the function of a mutated Usherin protein. The method may comprise providing a nucleic acid molecule as described in the present application, a vector as described in the present application, a viral particle as described in the present application, a cell as described in the present application, a pharmaceutical composition as described in the present application, and/or a kit as described in the present application. For example, by the splice-skipping of exon 13, the Usherin protein prepared in the present application maintains or substantially maintains the function of wild-type Usherin proteins in healthy individuals. For example, symptoms of retinitis pigmentosa, congenital degenerative nerve deafness, vestibular dysfunction, and other diseases are alleviated in subjects expressing the mutated Usherin protein.

In an aspect, the present application provides the use of a nucleic acid molecule as described in the present application, a vector as described in the present application, a cell as described in the present application, a pharmaceutical composition as described in the present application, and/or a kit as described in the present application in preparing a medicine for the prevention and/or treatment of diseases caused by USH2A gene mutations. For example, the diseases may comprise oculopathy and/or otopathy. For example, the disease may comprise Usher syndrome. For example, the disease may comprise type II Usher syndrome.

In an aspect, the present application provides a method for preventing and/or treating diseases caused by USH2A gene mutations. The method may comprise administering a nucleic acid molecule as described in the present application, a vector as described in the present application, a cell as described in the present application, a pharmaceutical composition as described in the present application, and/or a kit as described in the present application. For example, the diseases may comprise oculopathy and/or otopathy. For example, the disease may comprise Usher syndrome. For example, the disease may comprise type II Usher syndrome.

In an aspect, the present application provides a nucleic acid molecule as described in the present application, a vector as described in the present application, a cell as described in the present application, a pharmaceutical composition as described in the present application, and/or a kit as described in the present application, for the prevention and/or treatment of diseases caused by USH2A gene mutations. For example, the diseases may comprise oculopathy and/or otopathy. For example, the disease may comprise Usher syndrome. For example, the disease may comprise type II Usher syndrome.

USH2A generally refers to a gene encoding Usherin. USH2A is located at 1q41, which spans over 800kb in the genome and encodes a large transmembrane protein, Usherin, anchored to the plasma membrane of retinal photoreceptors and inner ear hair cells, a component essential for cilia development and maintenance. In the retina, Usherin is an important part of the USH2 complex and is thought to play a role in stabilizing the outer segment of photoreceptors. USH2A has two subtypes, with the predominant subtype in retinal cells having 72 exons and a coding region length of about 15.6kb. The extracellular moiety of the Usherin protein contains many repetitive domains, including 10 laminin EGF-like (LE) domains and 35 fibronectin type 3 (FN3) domains. The exon 13 of human USH2A is 642 bp in length and encodes amino acids 723-936, which is 4 of the 10 LE domains in the Usherin protein.

Mutations in exon 13, exon 50, and intron 40 of the USH2A gene may trigger Usher syndrome. Over 1000 pathogenic mutations distributed throughout the USH2A gene have been identified so far, of which exon 13 is the most frequently mutated exon in the USH2A gene, accounting for about 35%. Mutations in exon 13 of the USH2A gene include c.2802T>G (p.Cys934Trp, the highest frequency mutation in Chinese patients), c.2299delG (p.E767SfsX21, the highest frequency mutation in European and American patients), c.2276G>T (amino acid change: p.C759F), C.2522C>A (p.S841Y), c.2242C>T (p.Gln748X), c.2541C>A (C847X), c.2761delC (Leu921fs) and C.2776C>T (p.R926C), C.2209C>T, C.2310delA, c.2391_2392deITG, c.2431A>T, C.2431_2432delAA, c.2440C>T, c.2525dup, C.2610C>A, C.2755C>T, C.2176T>C, C.2236C>G, c.2296T>C, C.2332G>T.

The USH2A coding region is about 15.6kb in length. Conventional gene therapy delivery methods (e.g., recombinant lentiviruses, recombinant adeno-associated viruses, etc.) are difficult to package such bulky coding sequences and thus difficult to treat by direct delivery of USH2A. Exon 12 of mouse USH2A is homologous to exon 13 of human USH2A, with a length of 642 bp. Removal of this exon does not result in a subsequent frameshift mutation. Studies have shown that the Usherin can still locate correctly and function normally after the knockdown of exon 12 of mouse USH2A. For exon 13 of human USH2A containing pathogenic mutations, skipping may occur by a series of means for treatment.

Genomic DNA was edited by the CRISPR/Cas system to delete exon 13 directly or disrupt sites associated with RNA splicing. The use of fragment deletions may present risks, such as chromosomal rearrangements, viral integration, reverse re-integration, and the high off-target possibility of long-term expression of the CAS system or two gRNAs-induced double cleavage based on a relatively large genomic background. By using a single base editor to modify the key bases of the splicing-related sites above, the exon skipping may also be promoted. However, the existing single base editors may not be able to be loaded with a single AAV vector and may not have a suitable gRNA near the splicing-related sites, limited by the PAM, editing window, and type of base conversion. Targeted interference on pre-mRNA splicing using antisense oligonucleotides (AONs) has a higher efficiency in promoting exon skipping. AONs may promote the co-skipping of exons 12 and 13 while promoting the skipping of exon 13. Some AON treatments may all result in double skipping. However, due to the fact that exon 12 is 196 bp in full-length, not an integer multiple of 3, its deletion may result in frameshift mutations and the inactivation of USH2A protein. Targeted interference on pre-mRNA splicing using U7 snRNA results in a higher efficiency in promoting exon skipping than AONs and increases the proportion of single skipping of exon 13, so that safety is ensured while efficiency is significantly improved. The effect found in the present application, i.e., the efficiency of the snRNA molecules targeting the regions of the present application in promoting exon skipping, is higher than that of AON molecules targeting the same target, which is not obvious in the known prior art. For example, AON molecules targeting Chrl: 216247218-216247241 in the art have significantly higher efficiency in promoting exon skipping than snRNA molecules targeting this region. There are small nuclear RNAs (snRNAs) in cells, which are major components of RNA spliceosomes during posttranscriptional processing in eukaryotes and are involved in the processing of mRNA precursors by binding to snRNP proteins. Small nuclear RNAs are about 100-215 nucleotides in length in mammals and are classified into 7 classes, numbered U1-U7 due to its abundance of U. The modified U7 snRNA is prepared by replacing the non-canonical Sm binding site of U7 snRNA with the consensus sequence derived from the major spliceosome U snRNPs and changing the histone binding sequence at the 5' of U7 snRNA into the complementary sequence of the gene to be modified, which can induce splice-skipping of a exon by targeting the exon.

Without intending to be bound by any theory, the following examples are intended merely to illustrate the nucleic acids, methods of preparation, uses, etc., of the present application and are not intended to limit the scope of the present application.

### EXAMPLES

### Example 1

### Synthesis of snRNAs

Synthesis of snRNA backbone. The wild-type U7 snRNA includes a stem-loop structure (scafford), a U7-specific Sm sequence (AAUUUGUCUAG, SEQ ID NO: 2), and a recognition domain (complementary to histone pre-mRNA). The U7 snRNA of the present application can be derived from the gene sequence of mouse wild-type U7 snRNA on NCBI (NCBI Reference Sequence: NR_024201.3), with replacing the U7-specific Sm binding site (AATTTGTCTAG, SEQ ID NO: 3) with an optimized consensus Sm sequence, namely SmOPT (AATTTTTGGAG, SEQ ID NO: 4; or AAUUUUUGGAG, SEQ ID NO: 5), replacing the original recognition domain at the 5' end of the SmOPT sequence with a recognition domain reverse complementary paired with the specific target site for USH2A pre-mRNA, and retaining the original U7 stem-loop structure sequence (CAGGUUUUCUGACUUCGGUCGGAAAACCCCU, SEQ ID NO: 6) at the 3' end of the SmOPT sequence.

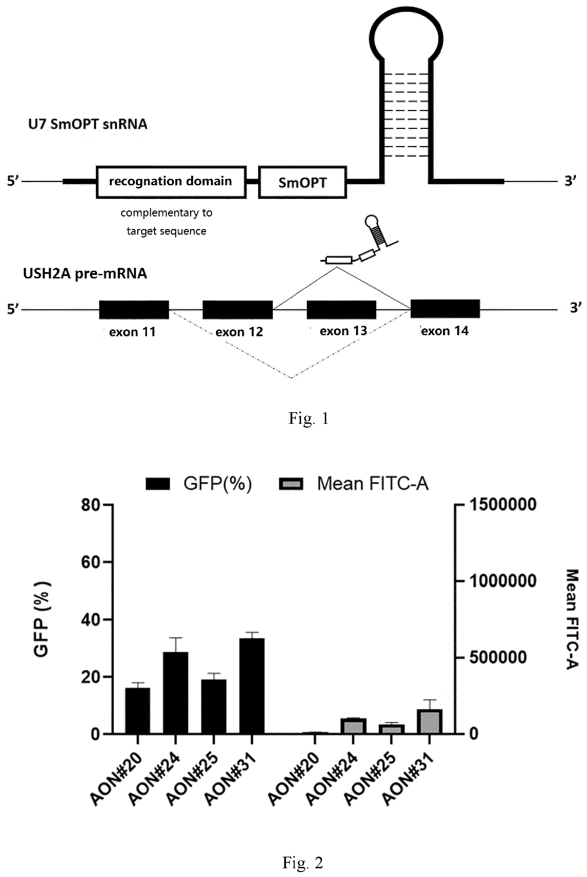

Fig. 1 is a schematic diagram of the structure and function of U7-snRNA according to the present application. The U7 snRNA recognition domain sequence for targeted induction of exon 13 of USH2A pre-mRNA is reverse complementary paired with a target sequence selected from intron 12-exon 13-intron 13 of USH2A pre-mRNA. The target sequence may be selected from the target region of the 3' sequence of exon 13 of USH2A pre-mRNA. The recognition domain sequence of snRNA is preferably 16 bp or more in length, further preferably 18 bp-40 bp in length, and still further preferably 20 bp-27 bp in length.

The specific operation process is as follows. First, a pUC57 vector containing a gene sequence: U7-snRNA gene expression cassette backbone (5'-mouse U7 promoter-smOPT sequence-U7 snRNA scafford-snRNA gene-specific 3' cassette-3'), was synthesized by means of total gene synthesis. Two Tpye IIs-type restriction enzyme recognition sites (e.g., BsaI, AarI, BsmBI, etc.) were added between the U7 promoter and smOPT to facilitate subsequent excision, substitution, and insertion of other recognition domain sequences. The snRNA gene-specific 3' cassette was a sequence behind the 3' end of the U7 snRNA gene in mouse genome (GenBank: X54748.1) comprising "GTCTACAATGAAA (SEQ ID NO: 7)", which was involved in the processing of pre-snRNA, preferably a gene fragment having a sequence length of 28-131 bp behind the 3' end of the U7 snRNA gene, further preferably a sequence length of 106 bp.

### Construction of U7 snRNA vectors targeting target regions

Corresponding Oligo DNAs were synthesized based on the pretranscriptional DNA sequences corresponding to snRNA recognition domain sequences in Table 1A, respectively. The sense strand of the Oligo DNAs was the DNA sequence corresponding to the recognition domain sequence, with CCGCA added at 5'. The antisense strand was the antisense complementary sequence of the recognition domain sequence, with AATT added at 5' and T added at 3'. For example, where the recognition domain sequence was 5'-NNN-3', the sense strand of the synthetic Oligo DNA was 5'*-CCGCA*NNN*-*3'*,* and the antisense strand was *5'-AATT*NNN*T-*3'*.*

The sense and antisense strands of synthetic Oligo DNAs were mixed according to an annealing reaction system (total reaction volume 20 µl: Oligo-F (100 µM) 2 µL + Oligo-R (100 µM) 2 µL + 10×NEB Cutter smart buffer 2 µL + deionized water 16 µL), incubated at 95°C for 5 minutes, and then placed on ice to cool and anneal to form double-stranded DNA with sticky ends. After 100-fold dilution, 1 µL was taken to ligate with 10 ng BsaI digested linearized pUC57-U7 snRNA backbone plasmid via T4 ligase. The ligation product was further transformed into E. coli competent cells, and single clones were selected. PCR and sequencing were performed to obtain a U7 snRNA vector for inducing the splice-skipping of exon 13 of USH2A. The plasmid was purified and stored at -20°C until use.

### Synthesis of AONs

Based on the target regions of exon 13 of USH2A pre-mRNAin Table 1B, sequences of antisense oligonucleotides (AONs) corresponding to the regions were designed and synthesized.

**Table 1A. The recognition domain sequences of snRNA**

| snRNA No. # | Recognition domain sequences (5'-3') | SEQ ID NO: | The genomic location corresponding to USH2A Pre-mRNA target sites |
|---|---|---|---|
| 4 | AUGAUCACACCUAAGCCCUAAAGAUAA | 36 | Chr1: |
| | | | 216247210-216247236 |
| 9 | AGAAAUUUAAAUCCAAAAUUGCAA | 8 | Chr1: |
| | | | 216247187-216247210 |
| 16 | ACACUGGCAGGGCUCACAUCCA | 37 | Chr1: |
| | | | 216247147-216247168 |
| 19 | ACUGAGCCAUGGAGGUUACACUGG | 9 | Chr1: |
| | | | 216247130-216247153 |
| 20 | AUUGUACCUGUGAGGCUCACACUG | 10 | Chr1: |
| | | | 216246730-216246753 |
| 24 | ACCUUCUUCCUUGACGAUUAGGCA | 11 | Chr1: |
| | | | 216246603-216246626 |
| 25 | AUUACACCUUCUUCCUUGACGAUU | 12 | Chr1: |
| | | | 216246598-216246621 |
| 26 | AUUACACCUUCUUCCUUGACGAUUAGG | 13 | Chr1: |
| | | | 216246598-216246624 |
| 27 | ACUGAUUACACCUUCUUCCUUGAC | 14 | Chr1: |
| | | | 216246594-216246617 |
| 28 | ACAUUUCUUUCUUACCUGGUUGAC | 15 | Chr1: |
| | | | 216246570-216246593 |
| 29 | ACAUUUCUUUCUUACCUGGUUG | 16 | Chr1: |
| | | | 216246570-216246591 |
| 30 | ACAUUUCUUUCUUACCUGGUUGACACU | 17 | Chr1: |
| | | | 216246570-216246596 |
| 31 | ACAUUUCUUUCUUACCUGGUUGCC | 18 | Chr1: |
| | | | 216246570-216246593 |
| 32 | ACAUUUCUUUCUUACCUGGUUGCCACU | 19 | Chr1: |
| | | | 216246570-216246596 |
| 33 | AAUACAUUUCUUUCUUACCUGGUU | 20 | Chr1: |
| | | | 216246567-216246590 |
| 34 | AUGUAAUACAUUUCUUUCUUACCU | 21 | Chr1: |
| | | | 216246563-216246586 |
| Scramble | AGGUGUAGUCGACCAUCGUG | 38 | No matching target sequence, negative control |

**Table 1B. The recognition sequences of AON**

| AON No. # | Sequences (5'-3') | SEQ ID NO: | The genomic location corresponding to USH2A Pre-mRNA target sites |
|---|---|---|---|
| 20 | mA*mU*mU*mG*mU*mA*mC*mC*mU* mG*mU*mG*mA*mG*mG*mC*mU*mC* mA*mC*mA*mC*mU*mG* | 10 | Chr1: |
| | | | 216246730-216246753 |
| 24 | mA*mC*mC*mU*mU*mC*mU*mU*mC* mC*mU*mU*mG*mA*mC*mG*mA*mU* mU*mA*mG*mG*mC*mA* | 11 | Chr1: |
| | | | 216246603-216246626 |
| 25 | mA*mU*mU*mA*mC*mA*mC*mC*mU* mU*mC*mU*mU*mC*mC*mU*mU*mG* mA*mC*mG*mA*mU*mU* | 12 | Chr1: |
| | | | 216246598-216246621 |
| 31 | mA*mC*mA*mU*mU*mU*mC*mU*mU* mU*mC*mU*mU*mA*mC*mC*mU*mG* mG*mU*mU*mG*mC*mC* | 18 | Chr1: |
| | | | 216246570-216246593 |

### Chemical synthesis and modification of U7-snRNA

Similar to oligonucleotides, U7 snRNA can also be synthesized by direct chemical synthesis to generate RNA containing a leader sequence, smOPT, and U7 snRNA scafford. U7 snRNA synthesized in vitro can be specifically modified to make it resistant to nuclease degradation or to increase affinity for target sequences.

In this example, U7 snRNA was chemically synthesized, with 2'-methoxy (2'-OME) and phosphorothioate modifications of the 3 bases at the 5' and 3' ends to increase nuclease resistance. Taking snRNA#25 and snRNA#26 as examples, chemically synthesized snRNA sequences and modifications are as follows (* indicates phosphorothioated backbone, m indicates 2'-methoxy modification, underlined indicates the recognition domain reverse complementary paired with the target sequence, italic indicates smOPT sequence):
Chemically synthesized and modified U7-snRNA#25: 
5'-mA*mU*mU*ACACCUUCUUCCUUGACGAUUAAUUUUUGGAGCAGGUUUU
CUGACUUCGGUCGGAAAACCmC*mC*mU*-3' (SEQ ID NO: 22)
Chemically synthesized and modified U7-snRNA#25 bidirectionally extended: 
5’-mA*mC*mU*GAUUACACCUUCUUCCUUGACGAUUAGGCAAAUUUUUGGA
GCAGGUUUUCUGACUUCGGUCGGAAAACCmC*mC*mU*-3' (SEQ ID NO: 23)
Chemically synthesized and modified U7-snRNA#26: 
5’-mA*mU*mU*ACACCUUCUUCCUUGACGAUUAGGAAUUUUUGGAGCAGGU

UUUCUGACUUCGGUCGGAAAACCmC*mC*mU*-3' (SEQ ID NO: 24)
Chemically synthesized and modified U7-snRNA#26 unidirectionally extended:

5’-mA*mU*mU*ACACCUUCUUCCUUGACGAUUAGGCACACACAGGCACAAU
UUUUGGAGCAGGUUUUCUGACUUCGGUCGGAAAACCmC*mC*mU*-3' (SEQ ID NO:
25)

One or more nucleotides in U7 snRNA of the present application are modified with one or more of 2'-O alkyl, 2'-O-methoxy, and 2'-O-methoxyethyl groups, preferably 2'-O alkyl is 2'-O-methyl modification. The phosphate bonds for nucleotide linkage of the snRNA can be specific phosphate bonds consisting of one or more of phosphorothioate bonds, phosphorodithioate bonds, alkyl phosphonate bonds, phosphoroamidate bonds, boranophosphate bonds, and chiral linkage phosphorus. In some embodiments, only 1-10, 6-80, or all of the nucleotides flanking a snRNA are modified, which is a combination of one or more modifications, and linked via specific phosphate bonds, which are a combination of one or more phosphate bonds. In some embodiments, all nucleotides in the chemically synthesized U7 snRNA are linked to each other via phosphorothioate bonds and modified with a 2'-O-methoxy group. In some embodiments, only 3 nucleotides flanking a snRNA are linked via phosphorothioate bonds and modified with a 2'-O-methoxy group. In some preferred embodiments of chemically synthesized and modified U7 snRNA, the first nucleotide at the 5' end of U7 snRNA is preferably adenylate (A). If the first nucleotide at the 5' end of the recognition domain is not adenylate (A), adenylate (A) is linked at the 5' end. In some embodiments of chemically synthesized and modified U7 snRNA, there may be 0-5 mismatched nucleotides, preferably 0-1, in the reverse complementary pairing between a recognition domain and a target site. The recognition domain sequence is preferably 16 bp or more in length, further preferably 18 bp-40 bp in length, and still further preferably 20 bp-27 bp in length. In some embodiments of chemically synthesized and modified U7 snRNA, the length of the recognition domain sequence may be further extended along the 5' or/and 3' ends of a target sequence by reverse complementary pairing, preferably the length of the extended single recognition domain sequence is less than or equal to 40 bp. In some embodiments, it is preferred that the chemically synthesized snRNA sequence has a total length greater than or equal to 96 bp. In some embodiments, it is preferred that 3-40 bases flanking the chemically synthesized snRNA are modified and linked via specific phosphate bonds.

### Example 2

Report vectors for quantitatively evaluating the efficiency of splice-skipping of exon 13 of USH2A

The RG_{left}-USH2A EXON13^{mut}-RG_{right} sequence (AgeI and EcoRI restriction sites were added at the 5' and 3' ends, respectively) was obtained by means of whole gene synthesis. The synthetic sequence and pX601 plasmid (Addgene, 61591) were subjected to restriction enzyme AgeI and EcoRI digestion, electrophoresis, gel recovery, and ligation. The synthetic sequence was inserted between the AgeI and EcoRI restriction sites of the pX601 vector, replacing the SaCas9 gene sequence of the original vector to obtain a reporter vector. The reporter vector was transformed into *E. coli* competent cells, and single clones were selected. PCR and sequencing were performed to obtain purified reporter vector plasmids, stored at -20°C until use.

The structure of the reporter vector was pCMV-RG_{left}-USH2A EXON13^{mut}-RG_{right}, where RG represents the reporter gene, RG_{left} represents the 5' first half of the reporter gene without reporting function, RG_{right} represents the 3' second half of the reporter gene without reporting function. The tandem expression of RG_{left} and RG_{right} can normally function as a complete reporter gene. In examples of the present invention, the reporter gene was the green fluorescent gene EGFP, and thus the vector structure was pCMV-EGFP_{left}-Exon13^{mut}-EGFP_{right}. EXON13^{mut} represents exon13 of USH2A containing pathogenic mutations, as well as upstream and downstream intron sequences thereof (the upstream intron sequence is the combined gene sequence of 204 bp at the 5' end and 490bp at the 3' end of intron 12 of human USH2Agene; the downstream intron sequence is the combined gene sequence of 703 bp at the 5' end and 216 bp at the 3' end of intron 13 of human USH2A). The pathogenic mutations in exon 13 of USH2A described in examples of the present invention may be c.2299delG or c.2802T>G or any mutation, and thus the vector structures obtained were pCMV-EGFP_{left}-Exon13^{c.2299delG}-EGFP_{right} and pCMV-EGFP_{left}-Exon13^{c.2802T>G}-EGFP_{right}, respectively. The mutations in some examples may also be or include c.2276G> T, C.2522C>A, c.2242C>T, c.2541C>A, c.2761delC, and C.2776C>T, etc.
RG_{left}, e.g. the sequence of EGFPi,ft is: 
atggtgagcaagggcgaggagctgttcaccggggtggtgcccatcctggtcgagctggacggcgacgtaaacggccacaa
gttcagcgtgtccggcgagggcgagggcgatgccacctacggcaagctgaccctgaagttcatctgcaccaccggcaagctgcccgtgc
cctggcccaccctcgtgaccaccctgacctacggcgtgcagtgcttcagccgctaccccgaccacatgaagcagcacgacttcttcaagtc
cgccatgcccgaaggctacgtccaggagcgcaccatcttcttcaaggacgacggcaactacaagacccgcgccgag (SEQ ID NO:
26)
RG_{right}, e.g. the sequence of EGFP_{right} is:

gtgaagttcgagggcgacaccctggtgaaccgcatcgagctgaagggcatcgacttcaaggaggacggcaacatcctggg

gcacaagctggagtacaactacaacagccacaacgtctatatcatggccgacaagcagaagaacggcatcaaggtgaacttcaagatccg

ccacaacatcgaggacggcagcgtgcagctcgccgaccactaccagcagaacacccccatcggcgacggccccgtgctgctgcccga

caaccactacctgagcacccagtccgccctgagcaaagaccccaacgagaagcgcgatcacatggtcctgctggagttcgtgaccgccg

ccgggatcactctcggcatggacgagctgtacaagtaa (SEQ ID NO: 27)

### Example 3

293 T cells were seeded into a 24-well plate in an amount such that the cell confluence reached approximately 80% after 24 hours. Then, 100 pmol synthetic antisense oligonucleotides AON#20, AON#24, AON#25, AON#31 (all nucleoside monomers of synthetic AON were subjected to 2'-O-methoxy and phosphorothioated modifications) were co-transfected into 293 T cells with the reporter plasmid pCMV-EGFP_{left}-Exon13^{mut}-EGFP_{right} using Lipofectamine2000, respectively. The transfected cells were further cultured for 48-72 hours, digested into single cells using trypsin, and then detected for the GFP positive rate (i.e., the proportion of cells in which exon 13 of USH2A pre-mRNA was induced for splice-skipping) and the mean FITC intensity of GFP positive cells (i.e. the average level of splice-skipping of exon 13 of USH2A pre-mRNA in GFP cells) in different AON groups using flow cytometry. Table 2 below and Fig. 2 show the effect of AONs targeting two regions to induce the splice-skipping of exon 13 of USH2A pre-mRNA. Experimental results show that AON targeting the 3' region has a significantly better effect than AON targeting other regions, such as region #20.

**Table 2. The effect of AONs targeting two regions to induce the splice-skipping of exon 13 of USH2A pre-mRNA.**

| Samples | GFP positive rate (%) | | | Mean FITC intensity | | |
|---|---|---|---|---|---|---|
| AON#20 | 15.77 | 14.77 | 18.33 | 14615.3 | 14648.4 | 16035.6 |
| AON#24 | 27.33 | 34.33 | 24.57 | 105370.5 | 108179.6 | 97134.6 |
| AON#25 | 21.07 | 19.78 | 16.95 | 78504.9 | 56500.1 | 64549.6 |
| AON#31 | 31.41 | 33.15 | 35.84 | 129221.5 | 132937.7 | 235885.3 |

### Example 4

293T cells were seeded into a 24-well plate in an amount such that the cell confluence reached approximately 80% after 24 hours. The pCMV-EGFP_{left}-Exon13^{mut}-EGFP_{right} and pUC57-U7 snRNA plasmids targeting USH2A pre-mRNA (with a vector mass ratio of 100 ng:400 ng) were co-transfected into 293 T cells using Lipofectamine2000. 293 T cells transfected with reporter plasmid alone (Report, reporter group) and co-transfected with reporter plasmid and pUC57-U7 Scramble (SC group) were used as two negative controls, respectively. 293 T cells not transfected with plasmids were used as a blank control. The transfected cells were further cultured for 48-72 hours, digested into single cells with trypsin, and then detected for the GFP-positive rate (i.e., the proportion of cells in which exon 13 of USH2A was induced for splice-skipping) in different U7 snRNA groups using flow cytometry. In this example, the mean FITC intensity in different experimental groups, i.e., the mean value of FITC fluorescence intensity of GFP-positive cells, as well as the GFP-positive rate and the expression amount of GFP protein in positive cells, were detected.

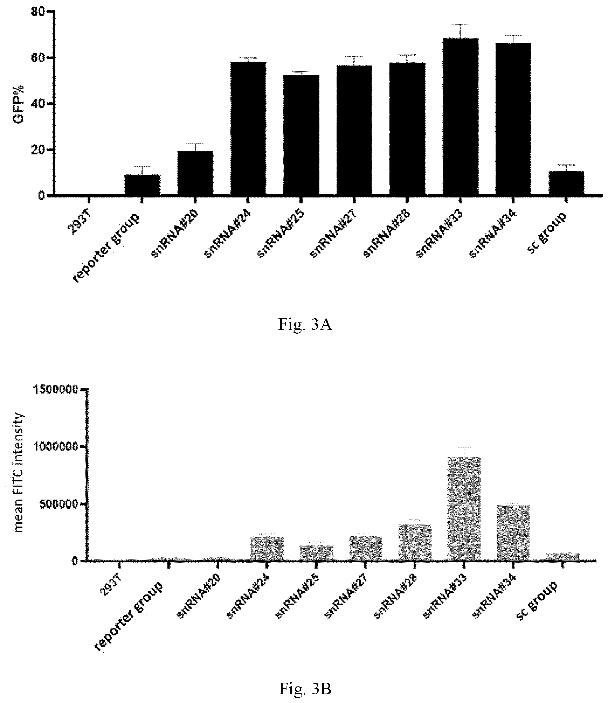

In this example, the efficiency of U7-snRNAs targeting different target sites to induce splice-skipping was compared. Table 3 below and Figs. 3A-3B show the effect of U7 snRNAs targeting different target sites to induce the splice-skipping of exon 13 of USH2A pre-mRNA in reporter cells. The results showed that all U7-snRNAs targeting the 3' sequence of exon 13 of USH2A pre-mRNA can induce the splice-skipping of exon 13 of USH2A in reporter cells, and the efficiency of USH2A to induce splice-skipping was high.

**Table 3. The proportion of cells in which the splice-skipping of exon 13 of USH2A pre-mRNA was induced in reporter cells by U7 snRNAs targeting different target sites.**

| Samples | GFP positive rate | | | Mean FITC intensity | | |
|---|---|---|---|---|---|---|
| | 1^{st} round | 2^{nd} round | 3^{rd} round | 1^{st} round | 2^{nd} round | 3^{rd} round |
| 293T | 0.1% | 0.1% | 0.1% | 6366 | 5100 | 8240 |
| reporter group | 9.2% | 12.8% | 6.1% | 23925 | 28917 | 17335 |
| snRNA#20 | 18.20% | 23.20% | 16.50% | 21260 | 18898 | 30766 |
| snRNA#24 | 55.70% | 59.10% | 59.30% | 204635 | 198488 | 239915 |
| snRNA#25 | 52.10% | 54.00% | 50.80% | 148755 | 110595 | 163122 |
| snRNA#27 | 52.90% | 61.00% | 55.70% | 222245 | 189021 | 243329 |
| snRNA#28 | 55.90% | 61.80% | 55.90% | 315629 | 283311 | 366659 |
| snRNA#33 | 64.10% | 75.20% | 66.40% | 819508 | 988642 | 918282 |
| snRNA#34 | 65.40% | 70.10% | 64.10% | 494047 | 473049 | 501321 |
| SC group | 11.00% | 13.30% | 7.70% | 58920 | 75279 | 66826 |

### Example 5

Efficient induction of single splice-skipping of exon 13 of USH2A pre-mRNA by chemically synthesized snRNAs

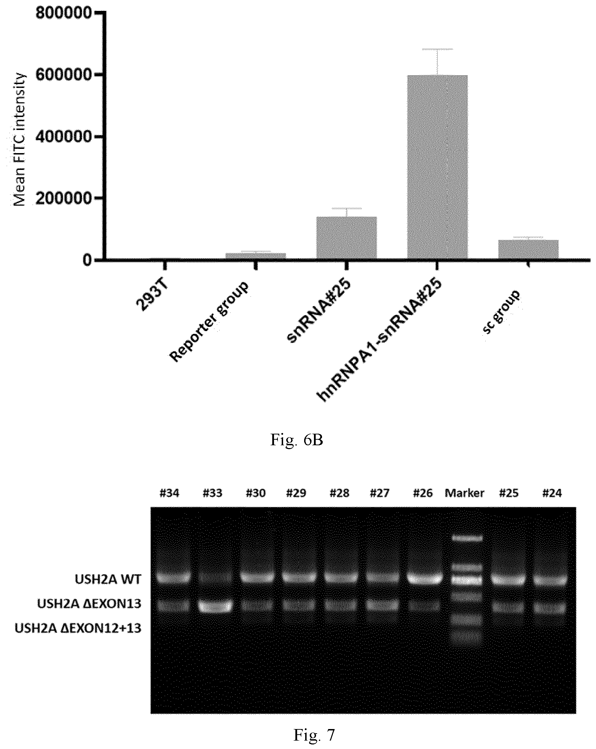

Human host cells were seeded at 6×10⁵/well into a 24-well plate. The human retinal nerve cells used in this example were WERI-Rb-1 cells (a retinal nerve cell line). 100 pmol U7-snRNA#24, #25, #26, #27, #28, #29, #30, #33, #34 synthesized in vitro were transfected into WERI cells using Lipofectamine2000. The transfected cells were further cultured for 72 hours. Next, RNA was extracted from each experimental group and reverse transcribed to obtain cDNA. RT-PCR experiments were performed by using primers AGCCTTTCCGCCAAGGTGATC (SEQ ID NO: 34) and CACAACGTTGCCCAGCAATGG (SEQ ID NO: 35) to detect whether the mature USH2A mRNA has splice-skipping of the exon. The electrophoresis results are shown in Fig. 7. The results showed that U7-snRNA#24-34 can efficiently induce the splice-skipping of exon 13, and almost no co-splice-skipping of exon 13 and exon 12 was observed. It can be concluded that U7 snRNA targeting the 3' region can efficiently induce the single splice-skipping of exon 13 of USH2A pre-mRNA with high safety.

### Example 6

The effect of splice-skipping of exon 13 of USH2A pre-mRNA mediated by U7-snRNA combinations targeting different target sites.

Construction of U7-snRNA multi-target combination vectors. According to the Golden Gate Assembly technique, using different U7 snRNA plasmids as templates, the U7 snRNA cassette (expression cassette) was amplified by PCR while introducing additional 5' flanking bases and BsaI restriction site in the correct orientation at both ends of the amplicon using primers, so that the adjacent different U7 snRNA cassettes were digested with BsaI to generate specific complementary sticky ends, and the first and last U7 snRNA cassettes were digested with BsaI to generate the same sticky ends as the linearized backbone vector digested by HindIII + NotI. Finally, the above PCR product was assembled with HindIII + NotI digested pUC57-U7 snRNA Backbone using NEB^{®} Golden Gate Assembly Kit (BsaI-HF^{®}v2) (NEB#E1601). The assembly method is as follows: pUC57-U7 snRNA Backbone-HindIII + NotI, 80 ng; U7 snRNA#A cassette PCR product, 20 ng; U7 snRNA#B cassette PCR product, 20 ng; U7 snRNA#C cassette PCR product, 20 ng; T4 DNA Ligase Buffer (10X), 2 µl; NEB Golden Gate Assembly Mix, 1 µl; reaction process: (37°C, 5 minutes → 16°C, 5 minutes) ×20 → 60°C, 5 minutes.

The Golden Gate Assembly product was further transformed into E. coli competent cells, and single clones were selected. PCR and sequencing were performed to obtain the U7 snRNA multi-target combination vector for inducing the splice-skipping of exon 13 of USH2A. The plasmid was purified and stored at -20°C until use. The vector constructed was exemplarily named pUC57-U7 snRNA#A + U7 snRNA#B + U7 snRNA#C.

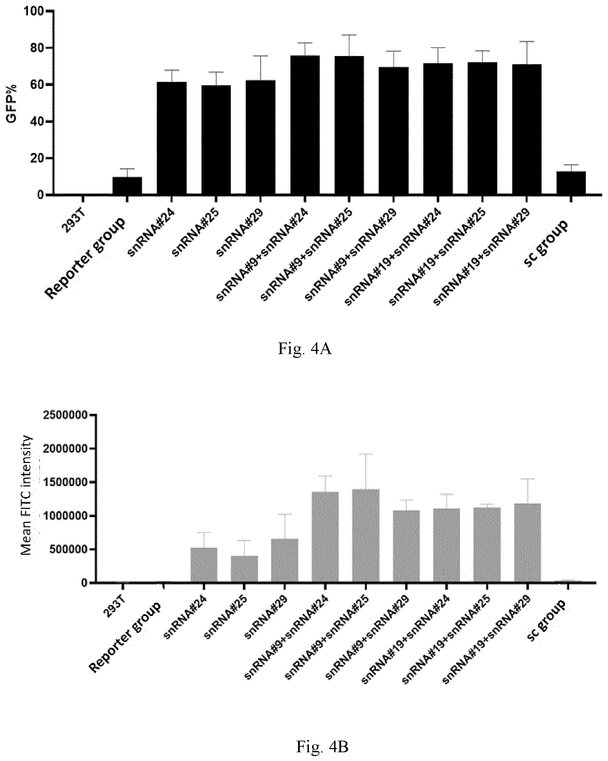

The U7-snRNA combinations targeting different targets more efficiently induce the splice-skipping of exon 13 of USH2A pre-mRNA. 293 T cells were seeded into a 24-well plate in an amount such that the cell confluence reached approximately 80% after 24 hours. The pCMV-EGFP_{left}-Exon13^{mut}-EGFP_{right} was co-transfected into 293 T cells with the U7 snRNA combination plasmids targeting different targets or single target plasmid (with a vector mass ratio of 100 ng:400 ng) using Lipofectamine2000, respectively. 293 T cells transfected with reporter plasmid alone (Report, reporter group) and co-transfected with reporter plasmid and pUC57-U7 Scramble (SC group) were used as two negative controls. 293 T cells not transfected with plasmids were used as a blank control. The transfected cells were further cultured for 48-72 hours, digested into single cells using trypsin, and then detected for the GFP-positive rate in different snRNA groups using flow cytometry. Table 4 below and Figs. 4A-4B show the efficiency of U7 snRNA combinations targeting different target sites to induce the splice-skipping of exon 13 of USH2A pre-mRNA.

In this example, by using U7-snRNA combinations targeting different target sites, it was found that the efficiency of inducing the splice-skipping of exon 13 of USH2A can be improved in reporter cells. The efficiency of inducing the splice-skipping by combinations of different targets in this example may be higher than that of a single target and may be superior to the known splice-skipping technique for exon 13 of USH2A.

**Table 4. The efficiency of U7 snRNA combinations targeting different target sites in inducing the splice-skipping of exon 13 of USH2A pre-mRNA.**

| Samples | GFP positive rate | | | Mean FITC intensity | | |
|---|---|---|---|---|---|---|
| | 1^{st} round | 2^{nd} round | 3^{rd} round | 1^{st} round | 2^{nd} round | 3^{rd} round |
| 293T | 0.10% | 0.10% | 0.10% | 5960 | 6100 | 11052 |
| Reporter Group | 5.10% | 11.00% | 13.50% | 19637 | 18264 | 25099 |
| snRNA#24 | 54.80% | 67.90% | 61.30% | 299833 | 495247 | 762459 |
| snRNA#25 | 52.00% | 66.60% | 60.00% | 200472 | 337127 | 656348 |
| snRNA#29 | 49.30% | 76.10% | 61.20% | 249725 | 969651 | 742796 |
| snRNA#9+snRNA#24 | 69.40% | 83.40% | 74.30% | 1122179 | 1355583 | 1590949 |
| snRNA#9+snRNA#25 | 66.30% | 88.30% | 72.10% | 908901 | 1954892 | 1307340 |
| snRNA#9+snRNA#29 | 64.20% | 79.50% | 65.10% | 972226 | 1256189 | 1011043 |
| snRNA#19+snRNA#24 | 63.60% | 80.40% | 71.10% | 873480 | 1148445 | 1297696 |
| snRNA#19+snRNA#25 | 69.70% | 79.20% | 67.50% | 1123782 | 1172973 | 1070130 |
| snRNA#19+snRNA#29 | 61.40% | 85.10% | 66.60% | 770099 | 1292896 | 1477626 |
| SC group | 8.60% | 14.40% | 15.40% | 37615 | 27557 | 41557 |

In the U7-snRNA combinations targeting different target sites of the present application, one of the snRNA can target the 3' sequence of exon 13 of USH2A pre-mRNA of the present application or a fragment thereof, and the other one or more snRNA can target the sequence of intron 12-exon 13-intron 13 of USH2A pre-mRNA or a fragment thereof. For example, U7-snRNA was reverse complementary paired with the target sequence of intron 12, exon 13, or intron 13 of USH2A pre-mRNA in a manner in terms of its recognition domain sequence, preferably the target sequence selected from exon 13 and flanking adjacent target regions (pre-mRNA region corresponding to chrl: 216246563-216247246). The U7-snRNA combinations targeting different target sites are combinations of two or more U7-snRNAs targeting not identical target sequences.

### Example 7

Induction of splice-skipping of exon 13 of USH2A pre-mRNA by chemically synthesized U7 snRNA combinations in WERI cells

Human host cells were seeded at 6×10⁵/well into a 24-well plate. The WERI-Rb-1 cell line was used for this example. 50 pmol snRNA combination 1 (U7-snRNA#30 and U7-snRNA#4) and combination 2 (U7-snRNA#26 and U7-snRNA#16) synthesized in vitro were transfected into WERI cells using Lipofectamine2000, respectively. The same dose (50 pmol) of antisense oligonucleotide AON1 (5'-MA*MG*MC*MU*MU*MC*MG*MG*MA*MG*MA*MA*MA*MU*MU*MU*MA*MA *MA*MU*MC*-3', "M" for 2'-O-methoxy modification, "*" for phosphorothioated) and AON2 (5'-MU*MG*MA*MU*MC*MA*MC*MA*MC*MC*MU*MA*MA*MG*MC*MC*MC*MU* MA*MA*MA*-3', "M" for 2'-O-methoxy modification, "*" for phosphorothioated) were transfected as control groups. 1 µg of EGFP plasmid was transfected as a negative control. WERI cells not transfected with any plasmids were used as a blank control. The transfected cells were further cultured for 72 hours. Next, RNA was extracted from each experimental group and reverse transcribed to obtain cDNA. RT-PCR experiments were performed by using primers AGCCTTTCCGCCAAGGTGATC (SEQ ID NO: 34) and CACAACGTTGCCCAGCAATGG (SEQ ID NO: 35) to detect whether the mature USH2A mRNA has splice-skipping of the exon. The electrophoresis results are shown in Fig. 8A. The rt-PCR bands were further quantitatively analyzed by ImageJ software, and the proportion of splice-skipping of exon 13 or splice-skipping of exons 12 and 13 of the mature USH2A mRNA was statistically analyzed, as shown in Fig. 8B.

In WERI cells endogenously expressing Usherin protein, the effect of U7 snRNA combinations targeting different target sites to induce the splice-skipping of exon 13 of USH2A pre-mRNA was compared with the preferred AON technical solution in the prior art. It can be seen from the RT-PCR test data and analysis results that the effect of snRNA combination 1 and snRNA combination 2 to induce the single splice-skipping of exon 13 is significantly superior to that of the preferred technical solutions AON1 and AON2 in the prior art, and the proportion of double splice-skipping of exons 12 and 13 of mRNA induced by snRNA combination 1 and snRNA combination 2 to the total splice-skipping of mRNA is lower than that of AON1 and AON2. Thus, it is clear that U7 snRNA significantly improves the efficiency of single splice-skipping of exon 13 while ensuring a lower by-product with double skipping of USH2A mRNA.

In addition, snRNA combination 2 is an AON site with a very high probability of double splice-skipping targeting the sequence adjacent to exons 12 and 13 in the prior art. However, snRNA combination 2 has a very low probability of double splice-skipping of the exons.

### Example 8

Effect of splice-skipping of U7 snRNA with motifs that may recruit splicing regulatory proteins

Construction of U7 snRNA linked to hnRNPA1 binding motif. Corresponding Oligo DNAs were synthesized based on the pretranscriptional DNA sequences corresponding to sequences in Table, respectively. The sense strand of the Oligo DNAs was the reverse complementary sequence of the target sequence (the DNA sequence corresponding to the recognition domain sequence), with CCGCAATATGA**TAGGGA**CT**TAGGGT**G (SEQ ID NO: 28) added at 5'. The antisense strand was the target sequence, with AATT added at 5' and C**ACCCTA**AG**TCCCTA**TCATATT (SEQ ID NO: 29) added at 3'. For example, in the case of the recognition domain sequence was NNN (the recognition domain was preferably greater than 16 nucleotides in length), the sense strand of the synthetic Oligo DNA was *CCGCAATATGA**TAGGGA**CT**TAGGGT**G*NNN, and the antisense strand was AATTNNN*C**ACCCTA**AG**TCCCTA**TCATATT* (underlined indicates the DNA double-stranded sequence corresponding to the recognition domain sequence, and bold italic indicates ***the DNA double-stranded sequence corresponding to the binding motif** "**UAGGGU*** (SEQ ID NO: 30)" ***of hnRNP A1 protein)**.*

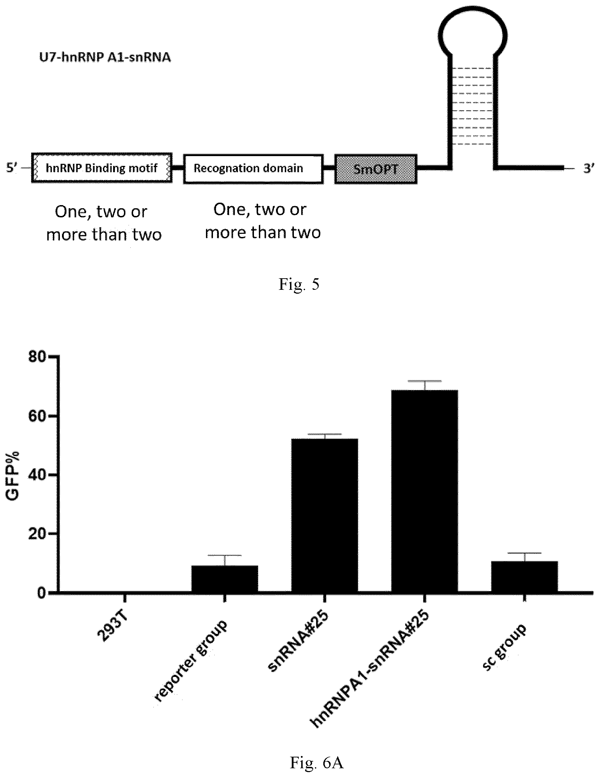

The sense and antisense strands of synthetic Oligo DNAs were mixed according to an annealing reaction system (total reaction volume 20 µl: Oligo-F (100 µM) 2 µL + Oligo-R (100 µM) 2 µL + 10×NEB Cutter smart buffer 2 µL + deionized water 16 µl), incubated at 95°C for 5 minutes, and then placed on ice to cool and anneal to form double-stranded DNA with sticky ends. After 100-fold dilution, 1 µL was taken to ligate with 10 ng BsaI digested linearized pUC57-U7 snRNA backbone plasmid. The product was transformed into E. coli competent cells, and single clones were selected. PCR and sequencing were performed to obtain a U7 snRNA vector containing the hnRNP A1 binding motif for inducing the splice-skipping of exon 13 of USH2A, named pUC57-U7-hnRNP A1-snRNA#A. The plasmid was purified and stored at -20°C until use. Fig. 5 is a schematic diagram of a snRNA vector with hnRNP A1.

U7-hnRNP A1-snRNA can also be chemically synthesized and modified according to the methods described in the examples of the present application. Taking snRNA#25 as an example, the chemically synthesized U7-hnRNP A1-snRNA sequence and modifications are as follows (* indicates phosphorothioated backbone, m indicates 2'-methoxy modification, underlined indicates the recognition domain reverse complementary paired with the target sequence, italic indicates *smOPT sequence,* bold indicates **the binding motif of hnRNP A1 protein):**

5'-mA*mC*mC*GCAAUAUGAUAGGGACUUAGGGUGAUUACACCUUCUUCC
UUGACGAUUAAUUUUUGGAGCAGGUUUCUGACUUCGGUCGGAAACC*mC*mC*
mU-3' (SEQ ID NO: 31).

U7 snRNA linked to the hnRNP A1 binding motif induced the splice-skipping of exon 13 of USH2A in reporter cells. 293 T cells were seeded into a 24-well plate in an amount such that cell confluence reached approximately 80% after 24 hours. The pCMV-EGFP_{left}-Exon13^{mut}-EGFP_{right} was co-transfected into 293 T cells with pUC57-U7-hnRNP A1-snRNA plasmid and pUC57-U7 snRNA plasmid (with a vector mass ratio of 100 ng:400 ng) using Lipofectamine2000, respectively. 293 T cells transfected with reporter plasmid alone (reporter group) and co-transfected with reporter plasmid and pUC57-U7 Scramble (SC group) were used as two negative controls. 293 T cells not transfected with plasmids were used as a blank control. The transfected cells were further cultured for 48-72 hours, digested into single cells using trypsin, and then detected for the efficiency of splice-skipping induced by different snRNA groups using flow cytometry. Table 5 below and Figs. 6A-6B show the efficiency of U7-hnRNP A1-snRNA to induce the splice-skipping of exon 13 of USH2A pre-mRNA.

The data show that the introduction of the hnRNP A1 binding motif at the 5' end of U7 snRNA can significantly improve the effect of inducing the splice-skipping of exon 13 of USH2A pre-mRNA, not only increasing the proportion of cells (GFP+) with splice-skipping of exon 13, but also increasing the level (mean FITC intensity) of mRNA splice-skipping the exon in each cell. At the same time, the introduction of the hnRNP A1 binding motif in U7 snRNA#25 resulted in a 3.25-fold increase in the average FITC strength and a significant improvement in the efficiency of inducing the splice-skipping.

**Table 5. The efficiency of U7-hnRNP A1-snRNA in introducing the splice-skipping of exon 13 of USH2A pre-mRNA.**

| Samples | GFP positive rate | | | Mean FITC intensity | | |
|---|---|---|---|---|---|---|
| | 1^{st} round | 2^{nd} round | 3^{rd} round | 1^{st} round | 2^{nd} round | 3^{rd} round |
| 293T | 0.1% | 0.1% | 0.1% | 6366 | 5100 | 8240 |
| reporter group | 9.2% | 12.8% | 6.1% | 23925 | 28917 | 17335 |
| snRNA#25 | 52.1% | 54.0% | 50.8% | 148755 | 110595 | 163122 |
| hnRNPA1-snRNA#25 | 67.9% | 72.1% | 66.3% | 680169 | 513447 | 603841 |
| SC group | 11.0% | 13.3% | 7.7% | 58920 | 75279 | 66826 |

In this example, a free tail was introduced at the 5' end of U7 snRNA. The free tail sequence includes the binding motif "UAGGGU" of hnRNP A1 protein and may contain one or more binding motifs of hnRNP A1 protein, preferably two. The free tail sequence is preferably "UAUGA**UAGGGA**CU**UAGGGU**G (SEQ ID NO: 32)", which can recruit hnRNP A1 protein, promote the splice-skipping of exon 13 of USH2A, and not increase the double skipping of exons 12 and 13, so that the targeting specificity thereof may not be affected, and the off-target effect may not be caused or increased. For example, the exon 12 of USH2A as described in the present application was exon 12 of human USH2A. For example, the exon 13 of USH2A as described in the present application was exon 13 of human USH2A.

In some embodiments, the free tail introduced at the 5' end of U7 snRNA is a motif that can recruit splicing regulatory proteins such as hnRNP A1 (Heterogeneous Nuclear Ribonucleoprotein A1), SRSF1 (Serine And Arginine Rich Splicing Factor 1), RBM4 (RNA Binding Motif Protein 4), DAZAP1 (DAZ Associated Protein 1), SR (Serine And Arginine-Rich Protein), etc.

### Example 9

Construction and viral packaging of AAV-U7 snRNA-related plasmid vector for targeted induction of splice-skipping of exon 13 of USH2A pre-mRNA

In this example, the U7 snRNA gene for targeted induction of splice-skipping of exon 13 of USH2A pre-mRNA was inserted into and replaced the gene sequence between two ITR domains of pAAV-CMV vector to construct pAAV-U7 snRNA vector. The pAAV-U7 snRNA vector was co-transfected into host cells with AAV packaging plasmid: serotype pRC plasmid (containing the AAV2 Rep gene and the respective Cap gene of each serotype) and pHelper plasmid (a vector plasmid containing the adenovirus E2A, E4 and VA genes), and packaged to obtain AAV-U7 snRNA virus for splice-skipping targeting exon 13 of USH2A pre-mRNA. The specific operation process is as follows.

First, a gene sequence-U7-snRNA gene expression cassette backbone (containing no recognition domain) was synthesized by means of total gene synthesis: 5'-mouse U7 promoter-smOPT sequence-U7 snRNA scafford-snRNA gene-specific 3' cassette-3'. Two Tpye IIs-type restriction enzyme recognition sites (e.g., BsaI, AarI, BsmBI, etc.) were added between the U7 promoter and smOPT to facilitate subsequent excision, substitution, and insertion of other recognition domain sequences. The sequence synthesized by means of total gene synthesis was inserted into and replaced the gene sequence between two AAV2-ITR domains of pAAV-CMV plasmid (AAVpro^{®} Helper Free System (AAV5) kit, TAKARA Corporation, Code No. 6650) to obtain the pAAV-U7 snRNA backbone vector.

Backbone of U7 snRNA gene expression cassette (without recognition domain) (SEQ ID NO: 33) :

AAGCTTAACAACATAGGAGCTGTGATTGGCTGTTTTCAGCCAATCAGCACTG
ACTCATTTGCATAGCCTTTACAAGCGGTCACAAACTCAAGAAACGAGCGGTTTTAATA
GTCTTTTAGAATATTGTTTATCGAACCGAATAAGGAACTGTGCTTTGTGATTCACATAT
CAGTGGAGGGGTGTGGAAATGGCACCTTGATCTCACCCTCATCGAAAGTGGAGTTGA

TGTCCTTCCCTGGCTCGCTACAGACGCACTTCCGCAgagaccaatgtgggtctcgAATTTTTGGA
GCAGGTTTTCTGACTTCGGTCGGAAAACCCCTCCCAATTTCACTGGTCTACAATGAAA
GCAAAACAGTTCTCTTCCCCGCTCCCCGGTGTGTGAGAGGGGCTTTGATCCTTCTCTG
GTTTCCTAGGAAACGCGTATGTGCGGCCGC

According to the method described in the above examples, the sense and antisense strands of corresponding Oligo DNAs were synthesized based on the pretranscriptional DNA sequences corresponding to the snRNA recognition domain sequences or the tandem combinations of recognition domain sequences of the present application, respectively, and both ends were added with sticky ends similar to those generated by the cleavage of Tpye IIs-type restriction enzyme recognition sites. The products were annealed to form a recognition domain (single/tandem) double-stranded DNA with sticky ends and then ligated into a linearized pAAV-U7 snRNA backbone plasmid digested with a corresponding Tpye IIs-type restriction enzyme via T4 ligase to form a pAAV-U7 snRNA plasmid targeting exon 13 of USH2A pre-mRNA for inducing splice-skipping. The plasmid was named based on the corresponding snRNA number of the recognition domain sequence, such as pAAV-U7 snRNA#25.

The gene of interest (U7-snRNA gene expression cassette for targeted induction of splice-skipping of exon 13 of USH2A pre-mRNA) was inserted into and replaced the gene sequence between AAV2-ITR domains of pAAV-CMV plasmid to obtain the pAAV-U7 snRNA plasmid vector. According to the instructions of AAVpro^{®} Helper Free System (AAV5) kit and the standard cell operating procedures, the plasmid vector was packaged to obtain the AAV-U7 snRNA virus for targeted induction of splice-skipping of exon 13 of USH2A pre-mRNA.

Twenty-four hours before transfection, HEK293/293T cells were seeded into a 100 mm cell-culture dish in a DMEM medium containing 10% FBS. Transfection was performed when the confluence reached 80%-90%. Three hours before transfection, the old medium was discarded and replaced with a fresh medium. At the time of transfection, pAAV-U7 snRNA plasmid, pRC plasmid, pHelper plasmid, and PEI (polyethylenimine) transfection reagent were prepared according to the following system and added dropwise to the culture dish. After adding the PEI transfection mixture, the culture dish was gently shaken to distribute the transfection reagent evenly and placed in a 37°C, 5% CO₂ incubator for culture.

PEI transfection system: pAAV plasmid (1 µg/µl), 6 µL; pRC1/2/5/6 plasmids (1 µg/µl); (pRC plasmid capsid gene determines serotype), 6 µL; pHelper plasmid (1 µg/µl), 6 µL; serum-free DMEM medium, 500 µL; PEI (1 mg/mL), 110 µL; treatment: vortex several times and incubate for 5 minutes at room temperature.

Twenty-four hours after transfection, a fresh DMEM medium containing 2% FBS was replaced. Forty-eight to 72 hours after transfection, cells containing AAV virus were harvested, washed, and centrifuged. The cell pellets were collected and vortexed to loosen. Subsequently, according to the instructions of AAVpro^{®} Helper Free System (AAV5) kit, 0.5 mL of AAV Extraction Solution A was added to the cell pellet and vortexed for 15 seconds to fully suspend the cell pellet. After standing for 5 minutes at room temperature, the mixture was vortexed for an additional 15 seconds. Cell debris was removed by centrifugation at 2000-14000g for 10 minutes at 4°C. The supernatant was collected into a new sterile centrifuge tube, and 50 µL AAV Extraction Solution B was added. The resulting mixture was mixed well using a pipette to obtain an AAV-U7 snRNA virus solution with different recognition domains. A part of the solution was taken to detect the virus titer using the qPCR method. The solution was stored at 80°C until use.

Since the gene fragment of interest inserted between AAV2-ITR domains of pAAV-U7 snRNA plasmid should be less than 2.5kb, it is feasible to insert multiple U7-snRNA gene expression cassettes (5'-mouse U7 promoter-smOPT sequence, U7 snRNA scafford-snRNA gene-specific 3' cassette-3'), so as to ensure that the expression level of U7 snRNA is increased under the condition of the same number of AAV virus particles. In the case of the gene sequence is about 450bp in length, it is preferred that the pAAV-U7 snRNA plasmid carries 1-5 U7-snRNA gene expression cassettes. The plurality of U7-snRNA gene expression cassettes in the pAAV-U7 snRNA plasmid may have the same recognition domain or combination of recognition domains or different or not identical combinations of recognition domains.

In the present application, U7 snRNA was delivered by AAV to induce the splice-skipping of exon 13 of USH2A pre-mRNA. The capsid protein of the AAV may be naturally derived, or a variant based on naturally-derived capsid protein, or undergo directed evolution or reasonable amino acid/peptide modification (codon optimization, chimerism of different serotype functional peptide segments, etc.) to improve the characteristics such as tissue and organ tropism, immunogenicity, and transfection efficiency, e.g., AAV2.5, AAV 2i8, AAV-TT, AAV9.HR, and CAM130. The naturally derived AAV capsid protein may be derived from animals or plants. The animal-derived AAV capsid protein may be derived from humans (such as AAV1, AAV2, AAV3, AAV5, AAV6, AAV7, AAV8, and AAV9), non-human primates (such as AAVrh.8, AAVrh.10, and AAVrh.43), vertebrates such as mouse and pig, or insects. In the AAV plasmid system of the present invention, for example, the AAV ITR serotype should be identical to the Rep gene serotype and different from the Cap gene serotype.

The foregoing detailed description is provided by way of explanation and example and is not intended to limit the scope of the appended claims. Various modifications to the embodiments presently recited in the present application will be apparent to those of ordinary skill in the art and are intended to be within the scope of the appended claims and their equivalents.

## Claims

1. A nucleic acid molecule having the ability to specifically bind to the 3' sequence of exon 13 of USH2A pre-mRNA or a fragment thereof, wherein the genomic location corresponding to the 3' sequence of exon 13 of USH2A pre-mRNA is Chrl: 216246563-216246753.

2. A nucleic acid molecule having the ability to specifically bind to the sequence of SEQ ID NO: 1 or a fragment thereof.

3. A nucleic acid molecule, wherein the nucleic acid molecule is complementary to 16 or more consecutive nucleotides of the sequence of SEQ ID NO: 1.

4. A nucleic acid molecule comprising 16 or more consecutive nucleotides of the sequence of SEQ ID NO: 1 or a complementary sequence thereof.

5. The nucleic acid molecule according to any one of claims 1-4, wherein the nucleic acid molecule comprises at least 16 nucleotides complementary to the 3' sequence of exon 13 of USH2A pre mRNA or the fragment thereof.

6. The nucleic acid molecule according to any one of claims 1-5, wherein the nucleic acid molecule comprises 22 to 27 nucleotides complementary to the 3' sequence of exon 13 of USH2A pre mRNA or the fragment thereof.

7. The nucleic acid molecule according to any one of claims 1-6, wherein the nucleic acid molecule comprises at least 16 nucleotides.

8. The nucleic acid molecule according to any one of claims 1-7, wherein the nucleic acid molecule comprises 22 to 27 nucleotides.

9. The nucleic acid molecule according to any one of claims 1-8, wherein the nucleic acid molecule comprises at least 60 nucleotides.

10. The nucleic acid molecule according to any one of claims 1-9, wherein the nucleic acid molecule comprises a sequence complementary to the pre-mRNA corresponding to genomic location selected from the following group: Chrl: 216246603-216246626, Chrl: 216246598-216246621, Chrl: 216246598-216246624, Chrl: 216246594-216246617, Chrl: 216246570-216246593, Chrl: 216246570-216246591, Chrl: 216246570-216246596, Chrl: 216246570-216246593, Chrl: 216246570-216246596, Chrl: 216246567-216246590, and Chrl: 216246563-216246586.

11. The nucleic acid molecule according to any one of claims 1-10, wherein the nucleic acid molecule comprises a sequence complementary to any one of SEQ ID NOs: 10-21.

12. The nucleic acid molecule according to any one of claims 1-11, wherein the nucleic acid molecule comprises a sequence of any one of SEQ ID NOs: 10-21.

13. The nucleic acid molecule according to any one of claims 1-12, wherein the nucleic acid molecule comprises a modified nucleotide.

14. The nucleic acid molecule according to claim 13, wherein the modified nucleotide comprises a modification selected from the following group: 2'-O-alkyl, 2'-O-methoxy, and/or 2'-O-methoxyethyl.

15. The nucleic acid molecule according to claim 13 or 14, wherein the modified nucleotide comprises a modification selected from the following group: 2'-O-methyl and/or 2'-O-ethyl.

16. The nucleic acid molecule according to any one of claims 13-15, wherein the nucleic acid molecule comprises at least one modified nucleotide.

17. The nucleic acid molecule according to any one of claims 13-16, wherein the 5' end of the nucleic acid molecule comprises at least one modified nucleotide.

18. The nucleic acid molecule according to any one of claims 13-17, wherein the 5' end of the nucleic acid molecule comprises 1 to 3 modified nucleotides.

19. The nucleic acid molecule according to any one of claims 13-18, wherein the 3' end of the nucleic acid molecule comprises at least one modified nucleotide.

20. The nucleic acid molecule according to any one of claims 13-19, wherein the 3' end of the nucleic acid molecule comprises 1 to 3 modified nucleotides.

21. The nucleic acid molecule according to any one of claims 1-20, wherein the nucleic acid molecule comprises a nucleotide or a nucleotide analog monomer selected from the following group: 6'-modified bicyclic nucleosides, 5'-modified bicyclic nucleosides, 6'-disubstituted bicyclic nucleosides, tetrahydropyran nucleoside analogs, and/or 2'-deoxy 2'-fluoro-β-D-arabinose nucleotides (2'-FANA modified nucleotides).

22. The nucleic acid molecule according to any one of claims 1-21, wherein the nucleic acid molecule comprises a modified phosphate bond.

23. The nucleic acid molecule according to any one of claims 1-22, wherein the nucleotide molecules are linked via the chemical bond selected from the following group: thiophosphate, phosphorodithioate, phosphotriester, alkyl phosphonate, aminoalkyl phosphotriester, alkylene phosphonate, phosphinate, phosphoramidate, aminoalkyl phosphoramidate, thiophosphoramidate, thiocarbonylalkyl phosphonate, thiocarbonylalkyl phosphotriester, phosphorothioate, selenophosphate, boranophosphate, phosphodiester bond, Rp-phosphorothioate bond, Sp-phosphorothioate bond, boranophosphate bond, methylene bond (methylimino), amide bond (3'-CH₂-CO-NH-5' and 3'-CH₂-NH-CO-5'), methylphosphonate bond, 3'-thiomethylal bond (3'S-CH₂-O5').

24. The nucleic acid molecule according to claim 22 or 23, wherein the modified phosphate bond is selected from the following group: thiophosphate bond, phosphorodithioate bond, alkyl phosphate bond, amide phosphate bond, and/or boranophosphate bond.

25. The nucleic acid molecule according to any one of claims 22-24, wherein the nucleic acid molecule comprises at least one modified phosphate bond.

26. The nucleic acid molecule according to any one of claims 22-25, wherein the 5' end of the nucleic acid molecule comprises at least one modified phosphate bond.

27. The nucleic acid molecule according to any one of claims 22-26, wherein the 5' end of the nucleic acid molecule comprises 1 to 3 modified phosphate bonds.

28. The nucleic acid molecule according to any one of claims 22-27, wherein the 3' end of the nucleic acid molecule comprises at least one modified phosphate bond.

29. The nucleic acid molecule according to any one of claims 22-28, wherein the 3' end of the nucleic acid molecule comprises 1 to 3 modified phosphate bonds.

30. The nucleic acid molecule according to any one of claims 1-29, wherein the nucleic acid molecule comprises DNA and/or RNA.

31. The nucleic acid molecule according to any one of claims 1-30, wherein the nucleic acid molecule is single-strand or double-strand.

32. The nucleic acid molecule according to any one of claims 1-31, wherein the nucleic acid molecule comprises an antisense oligonucleotide, shRNA, siRNA, miRNA, and/or aptamer.

33. The nucleic acid molecule according to any one of claims 1-32, wherein the nucleic acid molecule comprises a small nuclear RNA.

34. The nucleic acid molecule according to claim 33, wherein the small nuclear RNA comprises a U1 small nuclear RNA and/or U7 small nuclear RNA.

35. The nucleic acid molecule according to any one of claims 1-34, wherein the nucleic acid molecule does not bind to a Cas enzyme.

36. The nucleic acid molecule according to any one of claims 1-35, wherein the nucleic acid molecule does not comprise a structure capable of binding to a Cas enzyme.

37. The nucleic acid molecule according to claim 35 or 36, wherein the Cas enzyme is a Cas13 enzyme.

38. The nucleic acid molecule according to any one of claims 1-37, wherein the nucleic acid molecule comprises a stem-loop domain or a derivative structure thereof.

39. The nucleic acid molecule according to claim 38, wherein the stem-loop domain comprises the sequence of SEQ ID NO: 6.

40. The nucleic acid molecule according to any one of claims 1-39, wherein the nucleic acid molecule comprises a domain capable of binding to sm protein or a derivative structure thereof.

41. The nucleic acid molecule according to claim 40, wherein the domain capable of binding to sm protein comprises the sequence of SEQ ID NO: 5.

42. The nucleic acid molecule according to any one of claims 1-41, wherein the nucleic acid molecule comprises a domain capable of binding to a splicing regulatory protein.

43. The nucleic acid molecule according to any one of claims 1-41, wherein the nucleic acid molecule comprises a domain capable of binding to the protein selected from the following group: SRSF1 (Serine And Arginine Rich Splicing Factor 1), RBM4 (RNA Binding Motif Protein 4), DAZAP1 (DAZ Associated Protein 1), and SR (Serine And Arginine-Rich Protein).

44. The nucleic acid molecule according to any one of claims 1-41, wherein the nucleic acid molecule comprises a domain capable of binding to the hnRNP A1 protein.

45. The nucleic acid molecule according to claim 44, wherein the domain capable of binding to the hnRNP A1 protein comprises the sequence of SEQ ID NO: 30.

46. A gene expression cassette comprising or encoding the nucleotide sequence of the nucleic acid molecule according to any one of claims 1-45, and optionally an expression regulatory element.

47. A vector comprising or encoding the nucleotide sequence of the nucleic acid molecule according to any one of claims 1-45, and/or the nucleotide sequence of the gene expression cassette according to claim 46.

48. A viral particle comprising the nucleic acid molecule according to any one of claims 1-45, the nucleotide sequence of the gene expression cassette according to claim 46, and/or the vector according to claim 47.

49. A cell comprising the nucleic acid molecule according to any one of claims 1-45, the nucleotide sequence of the gene expression cassette according to claim 46, the vector according to claim 47, and/or the viral particle according to claim 48.

50. A pharmaceutical composition comprising the nucleic acid molecule according to any one of claims 1-45, the nucleotide sequence of the gene expression cassette according to claim 46, the vector according to claim 47, the viral particle according to claim 48, and/or the cell according to claim 49, and optionally a pharmaceutically acceptable carrier.

51. The pharmaceutical composition according to claim 50, wherein the pharmaceutical composition comprises a first vector comprising the nucleic acid molecule according to any one of claims 1-45, and a second vector comprising a nucleic acid molecule having the ability to specifically bind to exon 13 of USH2A pre-mRNA and flanking regions or a fragment thereof.

52. The pharmaceutical composition according to claim 50 or 51, wherein the flanking regions of exon 13 of USH2A pre-mRNA comprises intron 12 and/or intron 13 of USH2A pre-mRNA.

53. A kit comprising the nucleic acid molecule according to any one of claims 1-45, the nucleotide sequence of the gene expression cassette according to claim 46, the vector according to claim 47, the viral particle according to claim 48, the cell according to claim 49, and/or the pharmaceutical composition according to any one of claims 50-52.

54. A method for preparing the nucleic acid molecule according to any one of claims 1-45 comprising expressing and/or synthesizing the nucleic acid molecule capable of binding to 3' sequence of exon 13 of USH2A or a fragment thereof, wherein the genomic location of the 3' sequence of exon 13 of USH2A is Chrl: 216246563-216246753.

55. A method for inhibiting the expression and/or functioning of exon 13 of USH2A pre-mRNA, comprising providing the nucleic acid molecule according to any one of claims 1-45, the vector according to claim 47, the cell according to claim 49, the pharmaceutical composition according to any one of claims 50-52, and/or the kit according to claim 53.

56. A method for splice-skipping of exon 13 of USH2A pre-mRNA, comprising providing the nucleic acid molecule according to any one of claims 1-45, the vector according to claim 47, the cell according to claim 49, the pharmaceutical composition according to any one of claims 50-52, and/or the kit according to claim 53.

57. A method for preparing a mature USH2A mRNA with the deletion of exon 13, comprising providing the nucleic acid molecule according to any one of claims 1-45, the vector according to claim 47, the cell according to claim 49, the pharmaceutical composition according to any one of claims 50-52, and/or the kit according to claim 53.

58. A method for reducing the level of Usherin protein containing the expression product of exon 13, comprising providing the nucleic acid molecule according to any one of claims 1-45, the vector according to claim 47, the cell according to claim 49, the pharmaceutical composition according to any one of claims 50-52, and/or the kit according to claim 53.

59. A method for preparing a Usherin protein without the expression product of exon 13 and/or increasing the amount of Usherin protein without the expression product of exon 13, comprising providing the nucleic acid molecule according to any one of claims 1-45, the vector according to claim 47, the viral particle according to claim 48, the cell according to claim 49, the pharmaceutical composition according to any one of claims 50-52, and/or the kit according to claim 53.

60. A method for restoring the function of a mutated Usherin protein, comprising providing the nucleic acid molecule according to any one of claims 1-45, the vector according to claim 47, the viral particle according to claim 48, the cell according to claim 49, the pharmaceutical composition according to any one of claims 50-52, and/or the kit according to claim 53.

61. A use of the nucleic acid molecule according to any one of claims 1-45, the vector according to claim 47, the viral particle according to claim 48, the cell according to claim 49, the pharmaceutical composition according to any one of claims 50-52, and/or the kit according to claim 53 in preparing a medicament for the prevention and/or treatment of a disease caused by USH2A gene mutation.

62. The use according to claim 61, wherein the disease comprises an eye disease and/or ear disease.
